# EUROPEAN PATENT SPECIFICATION

(11) **EP 1 679 042 B1**
(45) Date of publication and mention of the grant of the patent: **14.06.2017**
(21) Application number: 04773197.1
(22) Date of filing: 16.09.2004
(51) Int. Cl.: A61B 17/28, A61B 18/14

(54) **SURGICAL INSTRUMENT**
CHIRURGISCHES INSTRUMENT
INSTRUMENT CHIRURGICAL

(30) Priority: 31.10.2003 JP 2003373607
(43) Date of publication of application: 12.07.2006
(73) Proprietor: Olympus Corporation, Tokyo 192-8507 (JP)
(72) Inventor: SEKINO, Naomi, Tokyo 192-8512 (JP)
(74) Representative: von Hellfeld, Axel
(86) International application number: PCT/JP2004/013550
(87) International publication number: WO 2005/041789

(56) References cited:
- EP-A2- 0 592 243
- JP-A- 6 197 906
- JP-A- 2003 144 451
- JP-A- 2003 144 451
- JP-A- 2003 235 856
- US-A- 5 391 180

## Description

### Technical Field

The present invention relates to a surgical instrument for carrying out surgery by manipulating a tool provided at a distal end of an insertion section at a manipulating section provided at a proximal end of the insertion section.

### Background Art

In Jpn. Pat. Appln. KOKAI Publication No. 2001-204734, an ultrasound surgical instrument is disclosed. In this instrument, vibration generated by a frontal ultrasonic transducer is transmitted to a probe formed of a metal material. A jaw openable with respect to a distal end of the probe is arranged at a distal end of this instrument. When ultrasonic transducer is transmitted to the probe while a living tissue is sandwiched between the jaw and the probe, the tissue is subjected to a coagulation procedure or an incision procedure by action of a frictional heat generated at the probe.

A heat exchange apparatus in which a grip section having a heating section at a distal end of the insertion section has been arranged is disclosed in a brochure WO01/12090A1. In this heat exchange apparatus, if a heating section is heated in a state in which a living tissue is sandwiched by a grip section, the living tissue is subjected to coagulation procedure or incision procedure. This grip section is formed in a bent shape. Thus, the grip section can be easily approached to a desired site of a living tissue.

A surgical forceps is disclosed in USP 5,643,294. This forceps includes a flexible tube at an elongated insertion section. When the flexible tube of the insertion section is bent in a desired direction by manipulating a manipulating section of the forceps, a pair of jaws provided at a distal end of the insertion section are curved in a desired direction together with the flexible tube of the insertion section. In this state, a pair of jaws can be opened and closed.

In the above-described ultrasound surgical instrument disclosed in Jpn. Pat. Appln. KOKAI Publication No. 2001-204734, it is necessary to form the probe in a linear shape in order to efficiently transmit an ultrasound wave to the probe. Thus, in a surgical operation using an endoscope, in particular, the ultrasound surgical instrument cannot be approached to a blood vessel or the like situated at the back of an organ, for example, or alternatively, are hardly approached, thus extending a time required for surgery.

The above-described heat exchange apparatus disclosed in a brochure of WO01/12090A1 has a shape such that the grip section at the distal end of the insertion section is bent or curved. However, in the case where this apparatus is used a heat exchange apparatus for endoscope operation in particular in order to insert the insertion section of the heat exchange apparatus through a trocar, it is difficult to form a bending shape of the grip section at the distal end of the insertion section in a sufficient bent shape because an inner diameter of the trocar maintained at a patent is restricted. EP 0 592 243 A2 discloses a device for performing endoscopic operations comprising an outer tube containing both an articulation tube and a drive rod. The drive rod moves in the longitudinal direction causing a gripping movement of the end effectors. The movement in the longitudinal direction of the articulation tube causes the end effector portion to deviate from the longitudinal axis. By rotating a rotation knob, it is also possible to rotate the outer tube together with the articulation tube and the drive rod.

JP 2003-144451 discloses a surgical device comprising, inter alia, a driving shaft, moveable by selecting a handle, so that the driving shaft moves longitudinally for actuating grasping jaws.

US 5,391,180 discloses a surgical instrument comprising jaws which are actuated through a cable that exerts a pulling action in a longitudinal direction. A deflection of the jaws is achieved by driving an elongated push rod.

### Disclosure of Invention

It is an object of the present invention to provide a surgical instrument capable of easily approaching a desired site of a living tissue and capable of making a coagulation procedure or an incision procedure of a living tissue. Claim 1 defines the scope of the invention and the dependent claims disclose the preferred embodiments.

The above object is achieved by a surgical instrument as defined in claim 1. According to an aspect of the invention, a surgical instrument includes: an insertion section having a distal end and a proximal end; a tool provided at the distal end of the insertion section, the tool having a turning section which can be turned in a direction deviating from an axial direction of the insertion section and a pair of grip sections which are relatively openable and; a manipulating section provided at the proximal end of the insertion section, the manipulating section having an opening-and-closing manipulating section which opens and closes the grip
sections and a turning manipulating section which turns the turning section; and a heating member which is arranged in at least one of the grip sections and which generates heat by power supply.

### Brief Description of Drawings

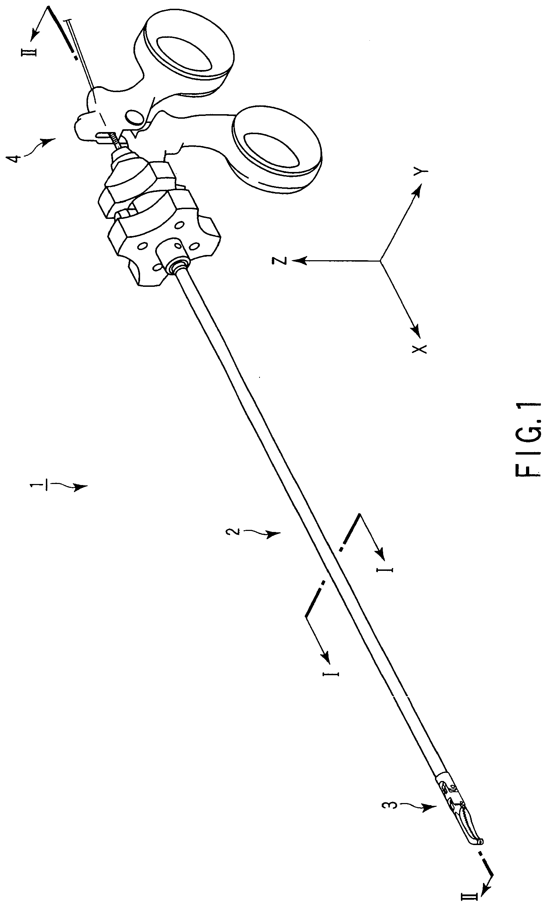

FIG. 1 is a perspective view schematically showing a whole structure of a surgical instrument according to a preferred embodiment of the present invention, wherein a surgery section is extended straightway to an axial direction of an insertion section and a state in which the surgery section is closed is seen from above;

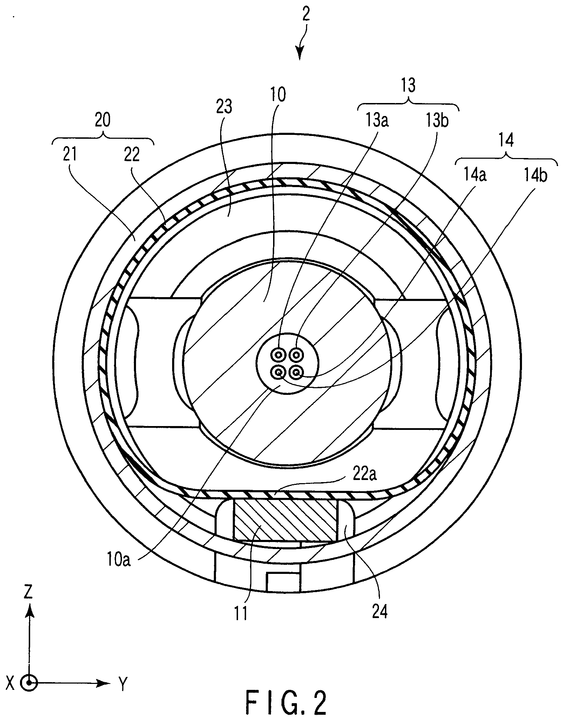

FIG. 2 is a sectional view taken along the line I-I in FIG. 1, showing an insertion section in a surgical instrument according to a preferred embodiment of the present invention;

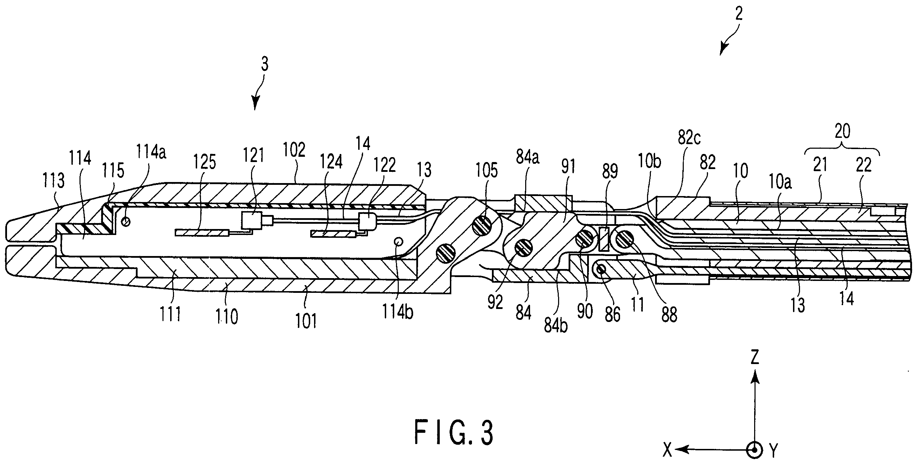

FIG. 3 is a sectional view taken along the line II-II in FIG. 1, showing a state in which a surgery section in the surgical instrument according to a preferred embodiment of the present invention is extended straightway in the axial direction of the insertion section and a state in which the surgery section is closed;

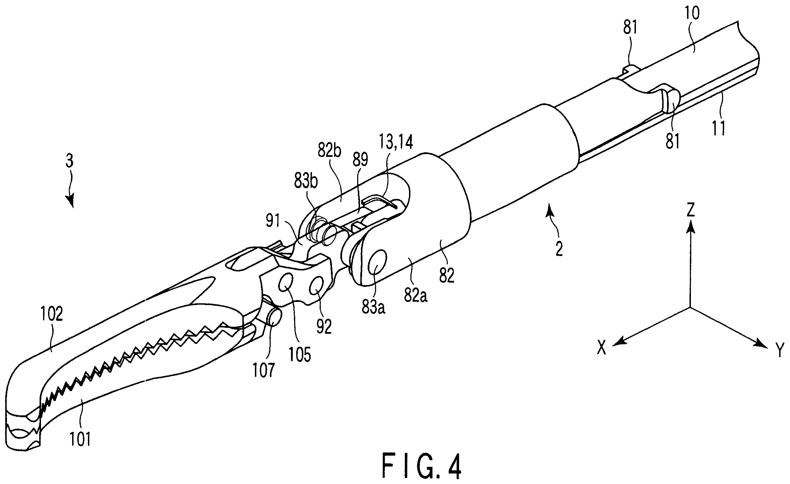

FIG. 4 is a perspective view when a state in which a sheath and a turning cover are removed is seen from above in a state in which the surgery section in the surgical instrument according to the preferred embodiment of the present invention is extended straightway in the axial direction of the insertion section and the surgery section is closed;

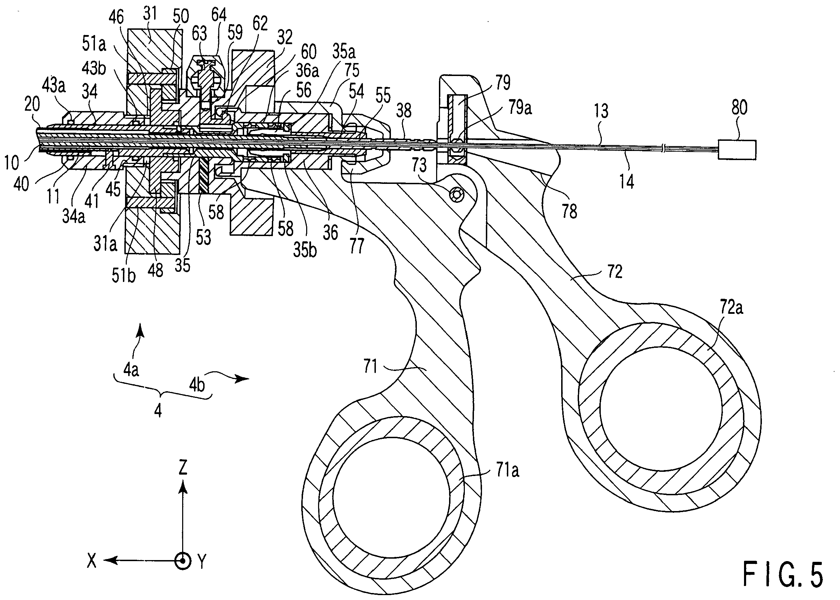

FIG. 5 is a sectional view taken along the line II-II in FIG. 1, showing a manipulating section in the surgical instrument according to a preferred embodiment of the present invention and showing a state in which the surgery section is extended in the axial direction of the insertion section and the surgery section is closed;

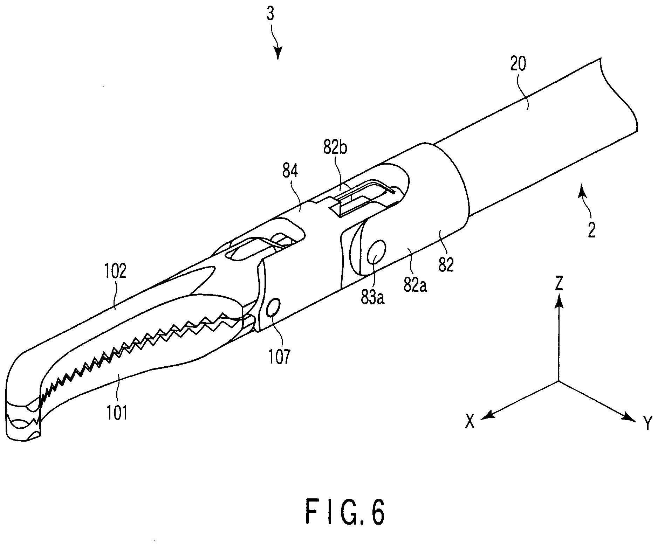

FIG. 6 is a perspective view when a state in which the surgery section in the surgical instrument according to a preferred embodiment of the present invention is extended straightway in the axial direction of the insertion section and the surgery section is closed is seen from above;

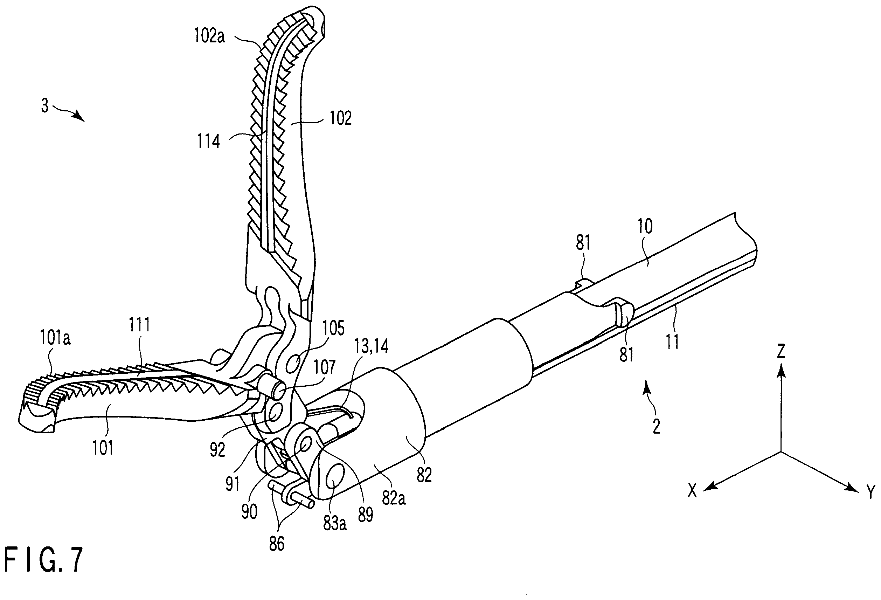

FIG. 7 is a perspective view when a state in which a sheath and a turning cover are removed is seen from above in a state in which the surgery section in the surgical instrument according to a preferred embodiment of the present invention is turned at 60 degrees with respect to the axial direction of the insertion section and the surgery section is opened to the maximum;

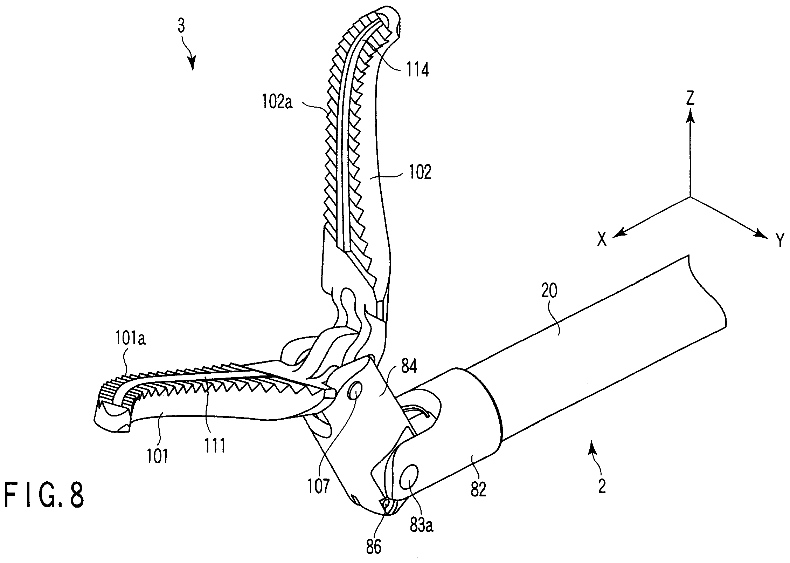

FIG. 8 is a perspective view when a state in which the surgery section in the surgical instrument according to a preferred embodiment of the present invention is turned at 60 degrees with respect to the axial direction of the insertion section and the surgery section is opened to the maximum is seen from above;

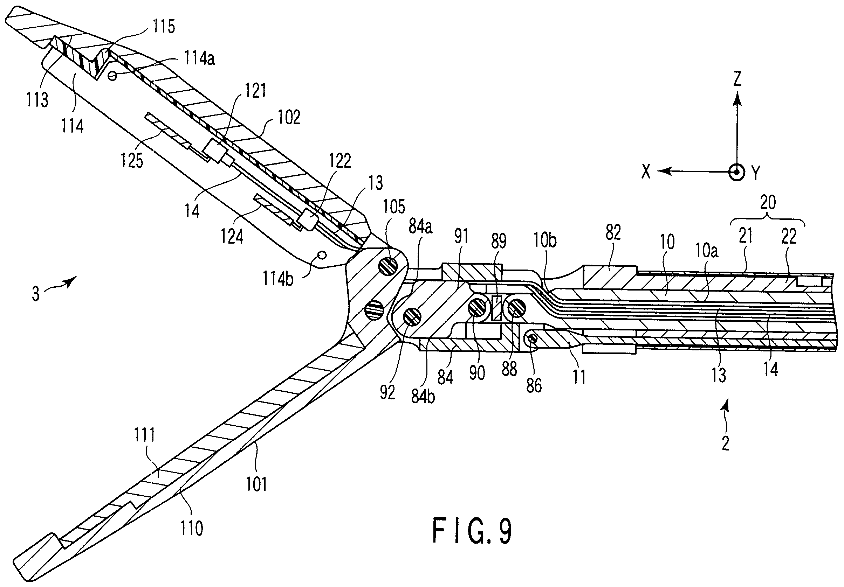

FIG. 9 is a sectional view taken along the line II-II in FIG. 1, showing a state in which the surgery section in the surgical instrument according to a preferred embodiment of the present invention is extended straightway in the axial direction of the insertion section and the surgery section is opened to the maximum;

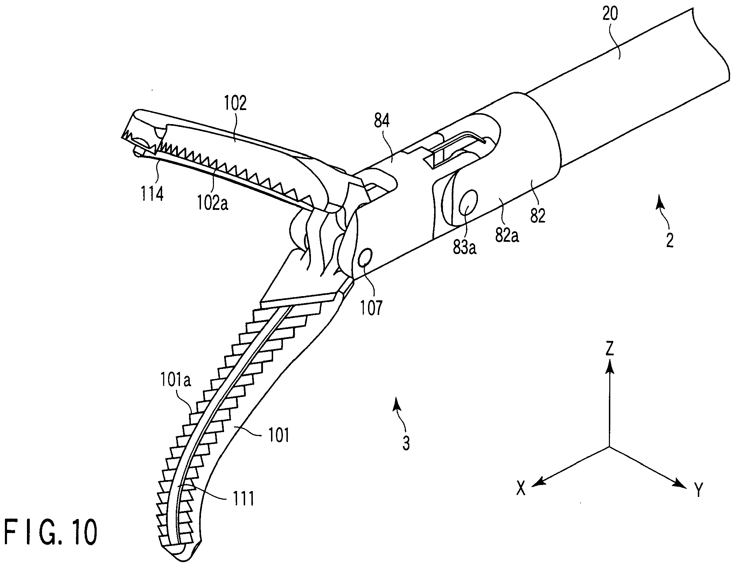

FIG. 10 is a sectional view when a state in which the surgery section in the surgical instrument according to a preferred embodiment of the present invention is extended straightway in the axial direction of the insertion section and the surgery section is opened to the maximum is seen from above;

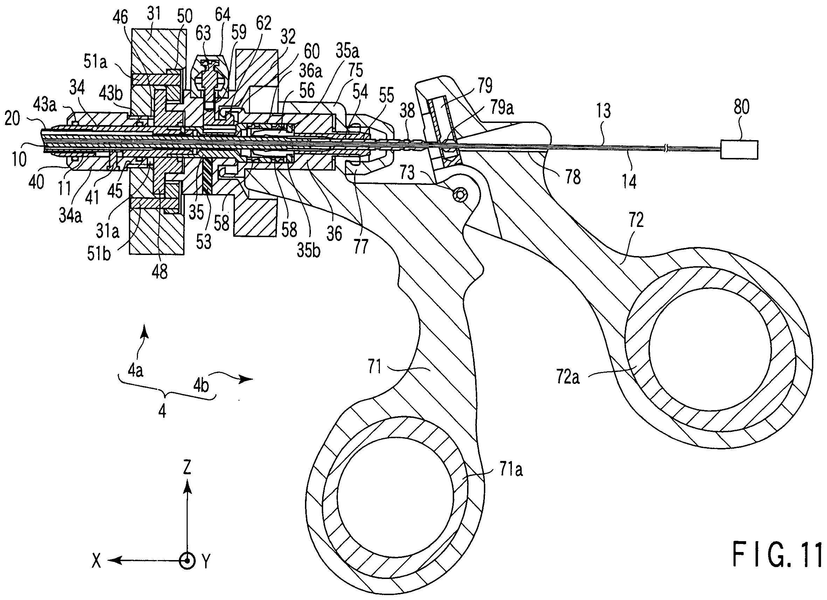

FIG. 11 is a sectional view taken along the line II-II in FIG. 1, showing a manipulating section in the surgical instrument according to a preferred embodiment of the present invention and showing a state in which the surgery section is extended straightway in the axial direction of the insertion section and the surgery section is opened to the maximum;

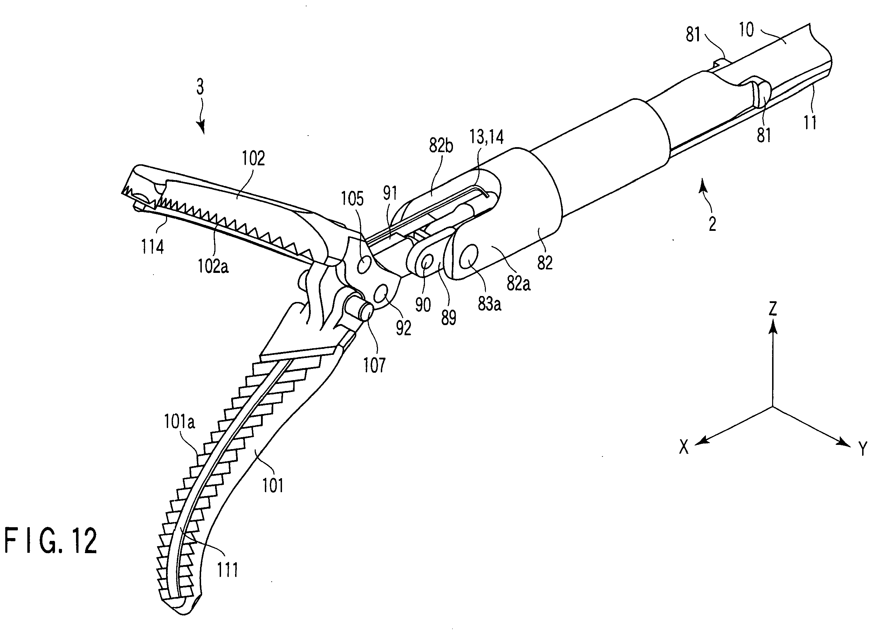

FIG. 12 is a perspective view when a state in which a sheath and a turning cover is removed is seen from above in a state in which the surgery section in the surgical instrument according to a preferred embodiment of the present invention is extended straightway to the axial direction of the insertion section and the surgery section is opened to the maximum;

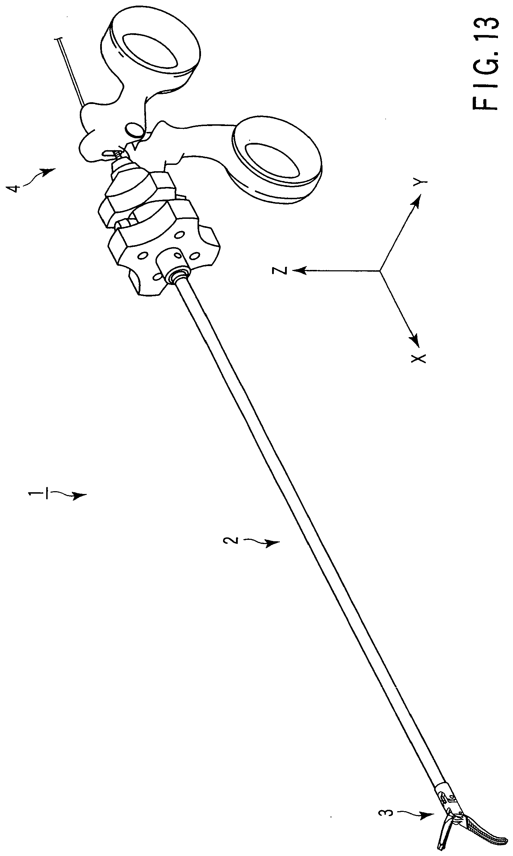

FIG. 13 is a perspective view schematically showing a whole structure of the surgical instrument according to a preferred embodiment of the present invention, wherein a state in which the surgery section is extended straightway in the axial direction of the insertion section and the surgery section is opened to the maximum is seen from above;

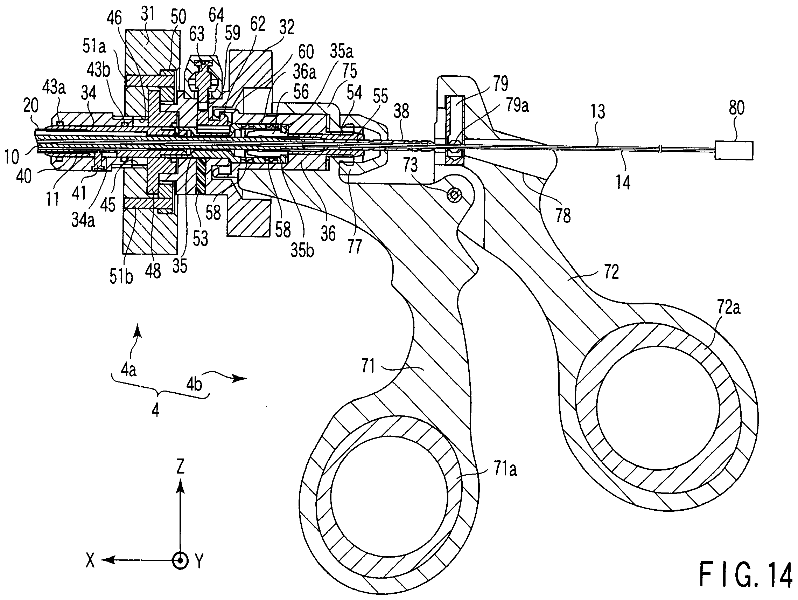

FIG. 14 is a sectional view taken along the line II-II in FIG. 1, showing a manipulating section in the surgical instrument according to a preferred embodiment of the present invention and showing a state in which the surgery section is turned at 60 degrees with respect to the axial direction of the insertion section and the surgery section is closed;

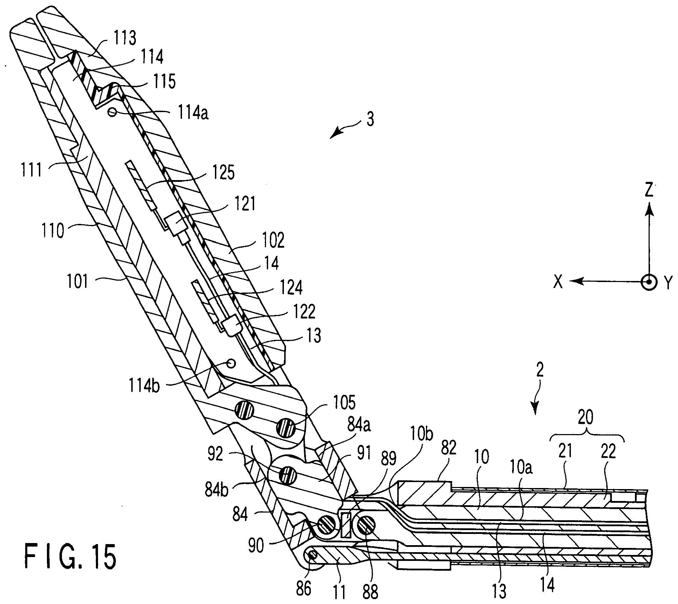

FIG. 15 is a sectional view taken along the line II-II in FIG. 1, showing a state in which the surgery section in the surgical instrument according to a preferred embodiment of the present invention is turned at 60 degrees with respect to the axial direction of the insertion section and the surgery section is closed;

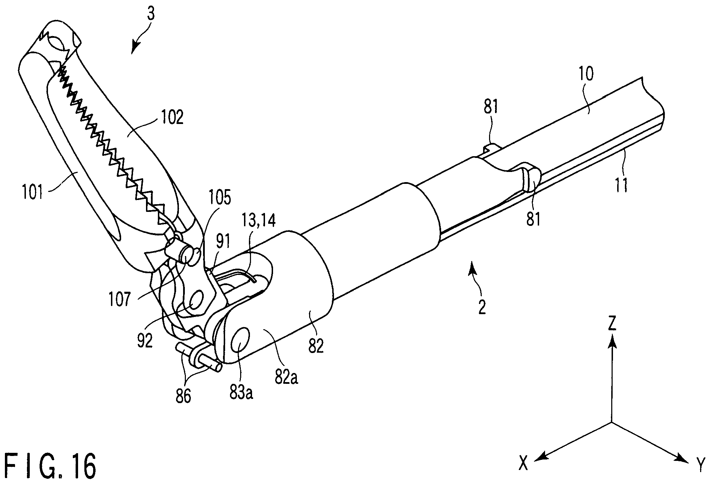

FIG. 16 is a perspective view when a state in which a sheath and a turning cover is removed is seen from above in a state in which the surgery section in the surgical instrument according to a preferred embodiment of the present invention is turned at 60 degrees with respect to the axial direction of the insertion section and the surgery section is closed;

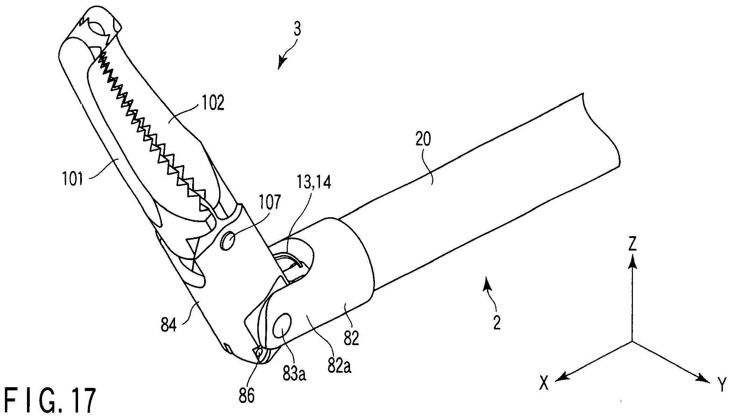

FIG. 17 is a perspective view when a state in which the surgery section in the surgical instrument according to a preferred embodiment of the present invention is turned at 60 degrees with respect to the axial direction of the insertion section and the surgery section is closed is seen from above;

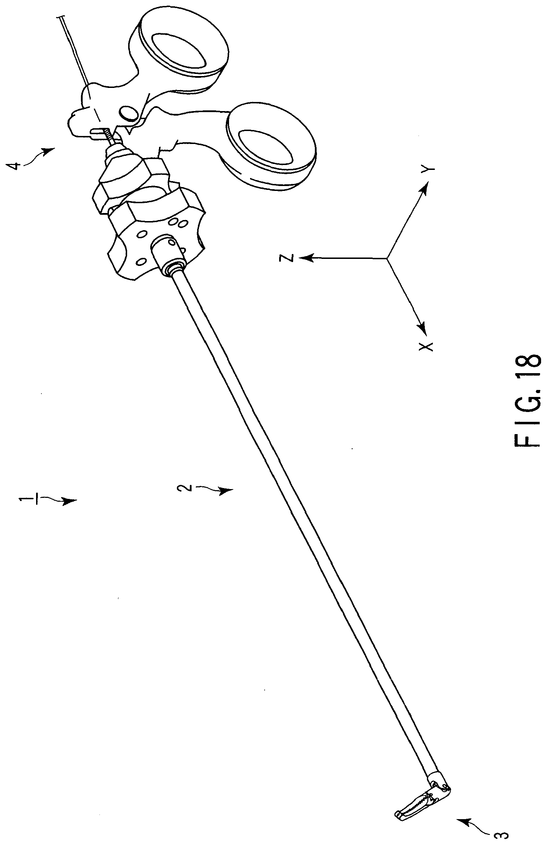

FIG. 18 is a perspective view schematically showing a whole structure of a surgical instrument according to a preferred embodiment of the present invention, wherein a state in which the surgery section is turned at 60 degrees with respect to the axial direction of the insertion section and the surgery section is closed is seen from above;

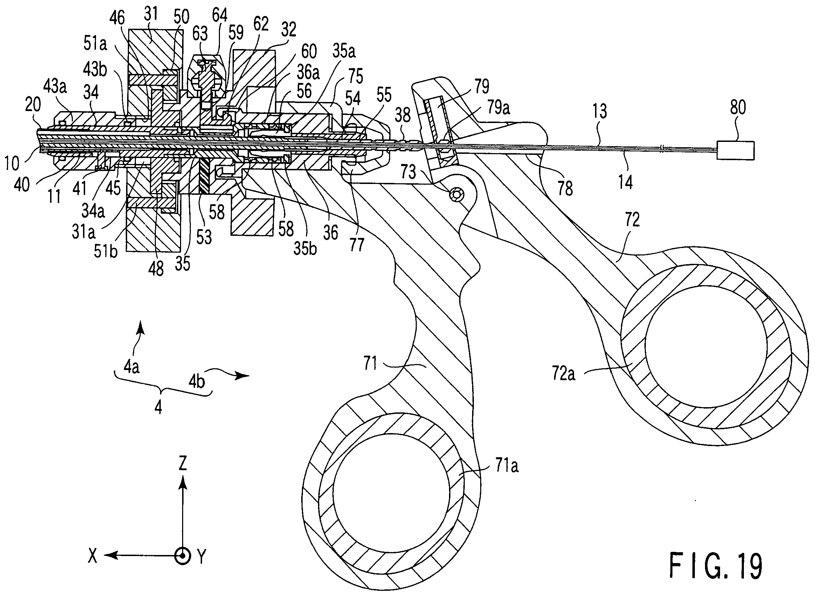

FIG. 19 is a sectional view taken along the line II-II in FIG. 1, showing a manipulating section in the surgical instrument according to a preferred embodiment of the present invention and showing a state in which the surgery section is turned at 60 degrees with respect to the axial direction of the insertion section and the surgery section is opened to the maximum;

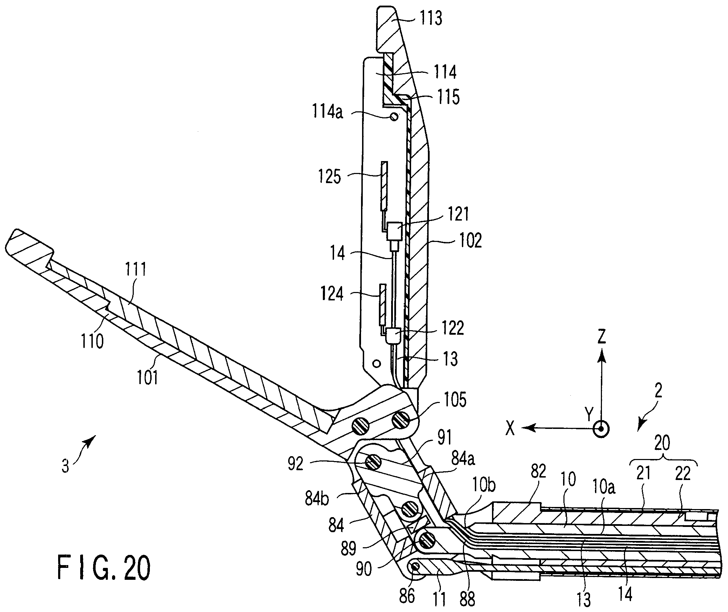

FIG. 20 is a sectional view taken along the line II-II in FIG. 1, showing a state in which the surgery section is turned at 60 degrees with respect to the axial direction of the insertion section and the surgery section is opened to the maximum; and

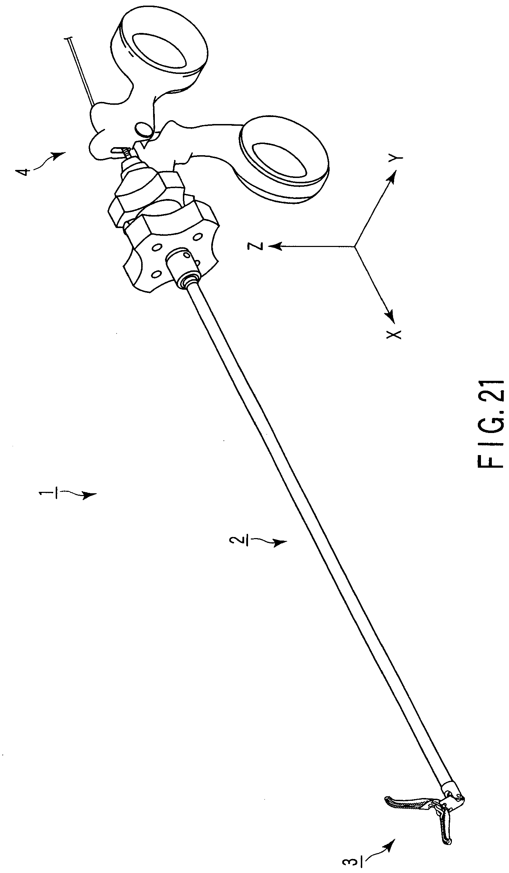

FIG. 21 is a perspective view schematically showing a whole structure of the surgical instrument according to a preferred embodiment of the present invention, wherein a state in which the surgery section is turned at 60 degrees with respect to the axial direction of the insertion section and the surgery section is opened to the maximum is seen from above.

### Best Mode for Carrying Out the Invention

Best mode for carrying out the present invention will be described with reference to the accompanying drawings.

As shown in FIG. 1, a surgical instrument 1 according to the present embodiment includes an insertion section 2, a treatment section 3, and a manipulating section 4. The insertion section 2 is formed in an elongated shape so as to be inserted into a cavity. The surgery section 3 is arranged at a distal end of the insertion section 2. The manipulating section 4 is arranged at a proximal end of the insertion section 2. When the manipulating section 4 is operated, the surgery section 3 is remotely operated through the insertion section 2.

As shown in FIGS. 2 to 4, at the insertion section 2, a first drive member (surgery section opening and closing actuator) 10 and a second drive member (surgery section turning actuator) 11 are disposed in parallel to or in substantially parallel to each other. The first drive member 10 and the second drive member 11 are provided as drive mechanisms for driving the surgery section 3 by manipulation of the manipulating section 4.

As shown in FIG. 2, the second drive member 11 has a transverse section formed as a rectangular bar body. The first drive member 10 is formed as a cylindrical tubular body, and its outer diameter is several millimeters. Thus, in the first drive member 10, an inner hole (third region) 10a is formed on a center axis of the first drive member 10. Two pairs of lead wires 13a, 13b, 14a, 14b (hereinafter, mainly designated by reference numerals 13, 14) insulated and covered with a heat resistance resin material such as PTFE, for example, are inserted into this inner hole 10a. These lead wires 13, 14 are extended to a further rear side than the manipulating section 4 on the center axis of the first drive member 10.

The first and second drive members 10, 11 are formed of a rigid body having rigidity equal to or greater than that capable of preventing buckling of the insertion section 2 when the insertion section is inserted into a living body and causes the surgery section 3 to make a turning manipulation or an opening and closing manipulation, thereby treating the living body. The first and second drive members 10, 11 are formed of a metal material such as a stainless steel material, for example, or a hard resin material such as a reinforced plastic material.

The first and second drive members 10, 11 are covered with a sheath (bulkhead) 20 having a double structure. The sheath 20 includes first and second sheaths 21, 22. The second sheath 22 is provided inside of the first sheath 21. A substantially full periphery of an outer periphery face of the first sheath 22 is brought into contact with an inner periphery face of the second sheath 21. The first and second sheaths 21, 22 are formed of a rigid body having rigidity equal to or greater than that capable of preventing buckling of the insertion section 2 when the insertion section is inserted into a living body and causes the surgery section 3 to make a turning manipulation or an opening and closing manipulation, thereby treating the living body. The first and second sheaths 21, 22 are formed of a metal material such as a stainless steel material, for example, or a hard resin material such as a reinforced plastic material.

A flat section (first flat section) 22a formed in a flat shape is formed partly of the peripheral face of the second sheath 22. A first lumen (first region) 23 is formed inside of the second sheath 22. The first drive member 10 is inserted into this first lumen 23. A second lumen (second region) 24 whose sectional area is smaller than that of the first lumen 23 is formed between the flat section 22a of the second sheath 22 and an inner periphery face of the first sheath 21. Thus, the flat section 22a of the second sheath 22 is formed as a bulkhead between the first lumen 23 and the second lumen 24. The second drive member 11 is inserted into the second lumen 24. The first and second drive members 10, 11 are arranged at the first and second lumens 23, 24, respectively, thus preventing interference of the first and second drive members 10, 11. Thus, the first and second drive members 10, 11 are retractable along the axial direction of the insertion section 2, respectively, independently.

The size of the second lumen 24 is formed to be slightly larger than a length in the thickness direction of the second drive member 11. As shown in FIG. 2, a traverse cross section of the second drive member 11 is formed in a rectangular shape. Thus, the second drive member 11 includes an abutment face (second flat section) which is slidably brought into face contact with the flat section 22a of the second sheath 22. The abutment face of the second drive member 11 is supported in a face contact state with the flat section 22a in the second lumen 24. A position opposed to the abutment face of the second drive member 11 is supported in a state abutted on an inner periphery face of the first sheath 21. Thus, even a member such as the second drive member 11 whose transverse section is formed in a rectangular shape and comparatively thinly, and extended to be elongated along the axial direction of the insertion section 2 is prevented from buckling when a living body is treated by means of the second lumen 24 having rigidity. Thus, a motive force is reliably transmitted toward the surgery section 3 by means of manipulation of the manipulating section 4 with respect to the second drive member 11.

As shown in FIG. 2, the first drive member 10 is arranged on the center axis of the insertion section 2. The lead wires 13, 14 arranged in the inner hole 10a of the first drive member 10 are arranged on the center axis of the insertion section 2. Thus, the first drive member 10 is movable along the axial direction on the center axis of the insertion section 2. The second drive member 11 is disposed eccentrically at the lower side shown in FIG. 2 with respect to the center axis of the first drive member 10 of the insertion section 2.

As shown in FIG. 5, the manipulating section 4 to be maintained and manipulated by a surgeon is arranged at a proximal end of the insertion section 2. This manipulating section 4 includes a turning manipulating section (tool orientation adjusting section) 4a and an opening-and-closing manipulating section (tool opening and closing section) 4b. The turning manipulating section 4a includes: a first rotary knob 31 for rotating the surgery section 3 in a predetermined range with respect to the axial direction of the insertion section 2 at a distal end of the insertion section 2, and a second rotation knob 32 for making rotation around the axis of the insertion section 2. The opening-and-closing manipulating section 4b includes first and second handles 71, 72 for opening and closing the surgery section 3.

The above first and second rotary knobs 31, 32 are arranged in parallel along the axial direction of the insertion section 2. The first rotary knob 31 is arranged at the distal end of the insertion section 2 rather than the second rotary knob 32. The first rotary knob 31 is rotatable in order to deflect the orientation of the surgery section 3 by rotating it around the distal end of the insertion section 2 (proximal end of the surgery section 3) with respect to the axial direction of the insertion section 2. The second rotary knob is rotatable in order to rotate the surgery section 3 around the axis of the insertion section 2.

These first and second rotary knobs 31, 32 comprise their respective cross shaped hook sections. Thus, the first and second rotary knobs 31, 32 are easily gripped and rotationally manipulated by a surgeon. The first and second rotary knobs 31, 32 are supported rotatably around an axis with respect to the center line of the insertion section 2 at the proximal end of the sheath 20 of the insertion section 2. A distal end of a rotation drive pipe 38 is engaged and coupled with the proximal end of the first drive member 10 while the lead wires 13, 14 are inserted. The rotation drive pipe 38 is extended to the further rear side than the first drive member 10.

The outer periphery face at the proximal end of the first sheath 21 of the sheath 20 is covered with a cylindrical first base 34. The proximal end of the first base 34 is extended to the further rear side than the proximal end of the sheath 20. Engagement sections are provided, respectively, at a proximal end of the first base 34 and at a distal end of a second base 35. These engagement sections are engaged with each other, and the first base 34 and the second base 35 are coupled with each other. A cylindrical third base 36 is rotatably coupled with the proximal end of the second base 35. A circular recess 36a having an inner periphery face with which the outer periphery face at the proximal end of the second base 35 is to be abutted is formed at the third base 36. A proximal end of the second base 35 is rotatably inserted into this recess 36a. These first to third bases 34, 35, 36 are formed in a cylindrical shape, respectively, and the first drive member 10 and a rotation drive pipe 38 are inserted.

Guide grooves 34a are formed in part of the vicinity of the distal end of the first base 34 described above and at the proximal end of the first sheath 21. The guide groove 34a penetrates from the outer periphery face of the first base 34 to the second lumen 24 and extends to a proper length along the axial direction of the insertion section 2. A turning base 40 is arranged at the outer periphery of the first base 34. A drive member fixing pin (adjusting section manipulating force transmitting means) 41 is attached to the turning base 40. The drive member fixing pin 41 is inwardly protruded through a through hole which penetrates from the outer periphery face to the inner periphery face of the turning base 40. An internal end of the drive member fixing pin 41 is coupled with a proximal end of the second drive member 11 through the guide groove 34a.

Recesses are provided, respectively, on the inner periphery faces of the distal end and proximal end of the turning base 40. At these recesses, for example, O-rings 43a, 43b are arranged as air tightness means. Thus, a pressure in an abdominal cavity is prevented from being lowered by the leakage of gas from the inside of the abdominal cavity from the distal end of the sheath 20 through the second lumen 24 and the proximal end of the first sheath 21 of the sheath 20 during surgical operation.

A male screw shaped first screw section 45 is formed on the outer periphery face of the proximal end of the turning base 40. The first rotary knob (surgery section turning manipulation rotary knob) 31 having a female screw shaped second screw section 45 on its inner periphery face is arranged so as to be spirally fitted to this first screw section 45. At the first rotary knob 31, a through hole 31a is formed on the center axis of the insertion section 2. From the distal end to the proximal end of the first rotary knob 31, the second screw section 46 is formed partly of the inner periphery face of the through hole 31a.

A step is formed at the through hole 31a of the first rotary knob 31. A diameter is formed to be constant as the second screw section 46 from the distal end to the proximal end of the through hole 31a of the first rotary knob 31 up to the intermediate position, and the diameter is extended at the proximal end of the second screw portion 46. At such a diameter-extended position, a flange 48 is arranged so as to be adjacent to the proximal end side of the second screw section 46. A ring shaped member 50 is arranged at the proximal end side of this flange 48. The first rotary knob 31 comes into contact with part of a distal end face of this ring shaped member 50 and the outer periphery face of the ring shaped member 50. In this state, the ring shaped member 50 is integrally fixed to the first rotary knob 31 by means of a plurality of bolts 51a, 51b.

A step is partially provided at a position at which the first rotary knob 31 and the flange 48 are opposed to each other so that a contact section between the first rotary knob 31 and the flange 48 is reduced to its required minimum in their mutual contact area. Thus, a contact area between the first rotary knob 31 and the flange 48 is reduced, and the generation of friction between the first rotary knob 31 and the flange 48 is kept low.

Similarly, at the contact section between the flange 48 and the ring shaped member 50, the flange 48 and the ring shaped member 50 are formed, respectively, so that their mutual contact area is reduced to its required minimum. Thus, the generation of friction between the flange 48 and the ring shaped member 50 is kept low. In this manner, the first rotary knob 31 can be easily rotated with respect to the flange 48. At this time, the first rotary knob 31 rotates in a state in which movement in the axial direction of the insertion section 2 is restricted. That is, the first rotary knob 31 is immobile forwardly and backwardly with respect to the axial direction of the insertion section 2.

The distal end of the second rotary knob 32 is arranged between the flange 48 and the ring shaped member 50. The distal end of this second rotary knob 32 is formed as an annular section. The annular section is arranged between the flange 48 and the ring shaped member 50. At the second rotary knob 32, as in the first rotary knob 31, through holes are formed around the center axis of the insertion section 2. At least part of the outer periphery face of the second base 35 is arranged so as to abut against the inner periphery face of the through hole of the second rotary knob 32. The second rotary knob 32 and the second base 35 are integrally coupled with each other by means of a fixing pin 53 orthogonal to the axial direction of the insertion section 2 and passing through the second rotary knob 32 and the second base 35.

A drive pipe cover member 54 is arranged at the outer periphery of an engagement section between the proximal end of the first drive member 10 and the distal end of the rotation drive pipe 38. Flat sections 55 are formed, respectively, at part of the outer periphery face of the rotation drive pipe 38 and part of the inner periphery face at the proximal end of the drive pipe cover member 54, and are integrated with each other by means of adhesive in a state in which the mutual flat sections 55 are abutted against each other.

At the distal end side of the drive pipe cover member 54, a guide section 56 is provided between the inner periphery face of the second base 35 and the outer periphery face of the first drive member 10. This guide section 56 receives rotating guide pins 35a, 35b provided at the second base 35. A pair of seal members 58 are arranged at this guide section 56. These seal members 58 support rotation of the second base 35 so as to be permitted with respect to the third base 36 and maintain a seal state inside of the second base 35.

Therefore, when the second rotary knob 32 is rotated around the axis of the insertion section 2, the second base 35 rotates together with the second rotary knob 32. In this manner, the drive pipe cover member 54 is axially rotated at the same time by means of the guide section 56. Thus, the rotation drive pipe 38 joined by means of the flat section 55 with the drive pipe cover member 54 rotates around the axis at the same time. The first drive member 10 coupled with the distal end of this rotation drive pipe 38 rotates around the center axis of the insertion section 2. The first base 34 engaged with the second base 35 by means of the engagement section also rotates following the first base 34. Thus, the turning base 40 also rotates around the center axis of the insertion section 2. Therefore, the sheath 20 also rotates following the first base.

The proximal end of the second base 35 and the outer periphery face of the drive pipe cover member 54 are covered with the third base 36. In the second rotary knob 32, a substantially L shaped recess 59 is formed at the outer periphery position of the insertion section 2. At the recess 59, an annular first engagement member 60 extending in the distal end direction of the insertion section 2 from the third base 36 is arranged. A hook shaped second engagement member 62 engaged with this first engagement member 60 is arranged at the second rotary knob 32. An engagement pin 63 and a push button 64 are provided at the second rotary knob 32. One end of the engagement pin 63 comes into contact at a distal side from a position engaged with the second engagement member 62 inside of the recess 59. The other end of the engagement pin 63 is protruded outside of the second rotary knob 32. The push button 64 covers the other end of the engagement pin 63, and is pressed by an operator. The push button 64 is formed of an elastic material such as a flexible rubber material, for example. The engagement pin 63 extends in a direction orthogonal to the axial direction of the insertion section 2. The first and second engagement members 60, 62 are engaged and disengaged from each other by pushing the push button 64 while the shape of the second engagement member 62 is maintained.

The second rotary knob 32 rotates around the axis of the insertion section 2. On the other hand, the third base 36 does not rotate. Thus, an engagement position of the second engagement member 62 changes depending on rotation around the axis of the insertion section 2 with respect to the first engagement member 60.

A turning manipulating section 4a is thus formed, and then, the first base 34 also rotates together with the second base 35 when the second rotary knob 32 is rotated. The first rotary knob 31 is also arranged at the outer periphery of the first base 34. Thus, when the second rotary knob 32 is rotated, the first rotary knob 31 also rotates together.

As described above, a contact section between the first rotary knob 31 and the flange 48 is formed so that their mutual contact area is reduced to its required minimum. Thus, the generation of friction between the first rotary knob 31 and the flange 48 is kept low.

A contact section between the flange 48 and the ring shaped member 50 is formed so that their mutual contact area is reduced to its required minimum. Thus, the generation of friction between the flange 48 and the ring shaped member 50 is kept low.

In the case where only the first rotary knob 31 is rotated while the second rotary knob 32 is in an immobile state, slippage occurs between the first rotary knob 31 and the flange 48 and slippage occurs between the flange 48 and the ring shaped member 50. Thus, only the first rotary knob 31 can be rotated around the axis of the insertion section 2 without using both hands while preventing rotation of the second rotary knob 32.

Now, an opening-and-closing manipulating section 4b provided at the proximal end of the insertion section 2 of the surgical instrument 1 will be described with reference to FIG. 5.

As shown in FIG. 5, the opening-and-closing manipulating section 4b maintained and manipulated by a surgeon includes first and second handles 71, 72. The first and second handles 71, 72 are formed of a resin material such as a hard plastic material, for example.

The first handle 71 is provided as a fixed handle. The first handle 71 is extended in a direction substantially orthogonal to the center axis of the insertion section 2. In the first handle 71, a finger hook ring 71a is formed at the lower end which is a position deviating from the center axis of the insertion section 2, thus reducing burden on fingers and hands of a manipulator.

The second handle 72 is pivoted by means of a handle pivoting pin 73 with respect to the first handle 71. The second handle 72 extends in obliquely downwardly and backwardly of the opening-and-closing manipulating section 4b rather than the first handle 71. In the second handle 72, a finger hook ring 72a is formed at the lower end which is a position deviating from the center axis of the insertion section 2, thus reducing burden on fingers and hands of a manipulator.

At the upper end of the first handle 71, a substantially cylindrical recess 75 is formed on an axis identical to the center axis of the insertion section 2. In the recess 75, the third base 36 is housed and fixed. From the inside to the outside, the lead wires 13, 14 sequentially covered with the rotation drive pipe 38 and the drive pipe cover member 54 further extend to the rear side through the center axis of the recess 75. The drive pipe cover member 54 has a proximal end at a position protruded at the slightly rear side than the proximal end of the recess 75. The rotation drive pipe 38 is extended to the further rear side than the proximal end of the recess 75. An air tight cap 77 maintaining air tightness at the proximal end of the recess 75 is attached to the proximal end of the recess 75. Thus, a pressure in an abdominal cavity is prevented from being lowered by the leakage of gas through the first and second lumens 23, 24 during a surgical operation.

At an upper end of the second handle 72, an inner hole 78 is formed on the center axis of the insertion section 2. The lead wires 13, 14 are inserted into this inner hole 78. A rotation drive pipe receptacle section 79 (opening and closing section manipulating force transmitting means) is arranged at the distal end of this inner hole 78. A ball section 79a is provided at this rotation drive pipe receptacle section 79. Thus, even if the second handle 72 is opened to be turned with respect to the first handle 71 around the handle pivoting pin 73, the ball section 79a rotates with respect to the rotation drive pipe receptacle section 79, and the lead wires 13, 14 can be received while they are retracted inside of the inner hole 78.

The opening-and-closing manipulating section 4b is thus formed. When the second handle 72 is opened or closed with respect to the first handle 71, the first drive member 10 can be moved forwardly and backwardly by means of the rotation drive pipe 38.

A connector 80 is provided at the proximal end of the lead wires 13, 14, and the connector 80 is electrically connected to a power supply device (not shown).

Now, the surgery section 3 provided at the distal end of the insertion section 2 of the surgical instrument 1 will be described with reference to FIGS. 3, 4, and 6 to 10.

An engagement receptacle section (not shown) is provided at the inner periphery face in the vicinity of the distal end of the second sheath 22. As shown in FIG. 3, an insertion section distal end cover 82 is provided at the inner periphery face of the distal end of the second sheath 22. A pair of engagement protrusions 81 (refer to FIG. 4) to be engaged with the engagement receptacle section is provided at the proximal end of the insertion section distal end cover 82. A large diameter section 82c is formed at the distal end of this insertion section distal end cover 82. This large diameter 82c is formed more frontally than the distal end of the first sheath 21 so that the distal end face of the first sheath 21 abuts. The large diameter section 82c is protruded more outwardly in a radial direction than the outer periphery face of the first sheath 21.

An opening end 10b of the inner hole (third lumen) 10a of the first drive member 10 is formed at the distal end of the first drive member 10. Thus, the lead wires 13, 14 are extended forwardly of the insertion section 2 from the opening end 10b and the distal end of the insertion section distal end cover 82.

As shown in FIGS. 4 and 6, a pair of distal end arms 82a, 82b protruded respectively to the distal end side of the insertion section 2 are formed at the insertion section distal end cover 82. The insertion section distal end cover 82 is formed of a rigid body having rigidity in the same manner as the second sheath 22. Thus, the insertion section distal end cover 82 is formed of a metal material such as a stainless steel material, for example, and a hard resin material (plastic material).

As shown in FIG. 6, at the distal end arms 82a, 82b of the insertion section distal end cover 82, a proximal end of a turning cover (joint section) 84 is pivoted by means of first turning pins 83a, 83b. Thus, the turning cover 84 is turnable while the first turning pins 83a, 83b at the distal end of the distal end arms 82a, 82b are defined as center axis (pivot axis). The turning cover 84 is formed of a rigid body having rigidity equal to or greater than that capable of preventing buckling of the turning cover 84 when the cover is inserted into a living body and causes the surgery section 3 to make a turning manipulation or an opening and closing manipulation, thereby treating a living tissue. Thus, the turning cover 84 is formed of a metal material such as a stainless steel material, for example, or a hard resin material such as a reinforced plastic material.

Now, the surgery section 3 in a state in which the turning cover 84 is removed will be primarily described with reference to FIGS. 4, 7, and 3 showing a cross section.

As shown in FIGS. 3, 7, and 8, a distal end of the second drive member 11 is pivoted at a proximal end of the turning cover 84 by means of a second turning pin 86. As shown in FIGS. 3 and 7, the second turning pin 86 is provided at the distal end of the second drive member 11. The second turning pin 86 is pivoted at a position which is offset with respect to a center line of the turning cover 84. Here, the second turning pin 86 is disposed at the lower side than the center line of the turning cover 84. The second turning pin 86 is provided in parallel to the first turning pins 83a, 83b described above. That is, the second turning pin 86 extends in a Y-axis direction.

As shown in FIG. 3, the distal end of the first drive member 10 is coupled with the proximal end of a first link arm (tool base) 89 by means of a first coupling pin 88 extending in the Y-axis direction. The distal end of the first link arm 89 is coupled with a proximal end of a second link arm (tool base, slide member) 91 by means of a second coupling pin 90 extending in the Y-axis direction. When first and second jaws 101, 102 described later are closed, the second coupling pin 90 is disposed on the same axis as the first turning pins 83a, 83b. When the first and second jaws 101, 102 described later are opened to the maximum, the first turning pins 83a, 83b and the first coupling pin 88 are disposed on the same axis.

These first and second link arms 89, 91 are formed of a rigid body having rigidity equal to or greater than that capable of preventing buckling when the arms are inserted into a living body and causes the surgery section 3 to make a turning manipulation or an opening and closing manipulation, thereby treating a living tissue. The first and second link arms 89, 91 are formed of a metal material such as a stainless steel material, for example, or a hard resin material such as a reinforced plastic material.

The second link arm 91 is slidable in a state in which the arm is abutted against two abutment faces 84a, 84b, internal periphery faces of a turning cover 84. The second link arm 91 is always abutted against the two abutment faces 84a, 84b so that the second link arm 91 always retracts in parallel to the turning cover 84.

In the vicinity of one abutment face 84a of the second link arm 91, an opening is provided at a side part of the second link arm 91 and the second link arm 91, and the lead wires 13, 14 are inserted into the turning cover 84. The distal ends of the lead wires 13, 14 are guided into a heat transmission member 114 of the second jaw 102 described later.

As shown in FIGS. 3 and 9, the distal end of the second link arm 91 capable of sliding in the inside of the turning cover 84 is pivoted by means of a third coupling pin 92. The third coupling pin 92 extends in the Y-axis direction in parallel to the proximal end (second coupling pin 90) of the second link arm 91. By means of the third coupling pin 92, the proximal end of the first jaw 101 is pivoted from among the first and second jaws (grip sections) 101, 102 which configure a distal end tool. Thus, the distal end of the second link arm 91 and the proximal end of the first jaw 101 are coupled with each other by means of the third coupling pin 92.

As shown in FIG. 4, the proximal end of the first jaw 101 is buckled in a direction deviating from the axial direction of the surgery section 3 forwardly from a position coupled with the third coupling pin 92. The first jaw 101 is formed to be bent so as to be parallel to the axial direction of the surgery section 3 again partway as it goes forward.

The second jaw 102 is supported at the proximal end of the first jaw 101. The second jaw 102 is pivoted at the proximal end of the first jaw 101 by means of a first opening and closing pin 105 which extends in the Y-axis direction. The proximal end of the second jaw 102 is buckled in a direction deviating from the axial direction of the surgery section 3 forwardly from a position coupled with the first opening and closing pin 105. The proximal end of the second jaw 102 is formed to be bent so as to be parallel to the axial direction of the surgery section 3 partway as it goes forward. A pair of jaws 101, 102 open and close the inside of a ZX plane, for example, substantially symmetrically with respect to the axial direction of the surgery section 3.

As shown in FIGS. 8 and 10, at the first and second jaws 101, 102, grip faces 101a, 102a are provided at positions opposed to each other symmetrically to the axial direction of the surgery section 3, respectively. Irregularities (slip-proof) are formed on these grip faces 101a, 102a as required.

One side face adjacent to the grip faces 101a, 101b of the first and second jaws 101, 102 is formed in substantially parallel to the ZX plane in the axial direction of the surgery section 3. The other side face adjacent to the grip faces 101a, 101b are formed to be curved so as to draw an arc in an XY plane from the proximal end to the distal end of the first and second jaws 101, 102. The distal end and proximal end of the side face adjacent to these grip faces 101a, 102a are smoothly formed.

A second opening-and-closing pin 107 is provided in the Y-axis direction at the proximal end of a site having the grip face 101a of the first jaw 101. As shown in FIG. 6, this opening-and-closing pin 107 is supported by the turning cover 84. The turning cover 84 specifies a distance between the first turning pins 83a, 83b and the second opening-and-closing pin 107. The second link arm 91 and the third coupling pin 92 (refer to FIG. 4) at a distal end of this second link arm 91 are slidably provided inside of this turning cover 84. Thus, the proximal end of the second jaw 102 is also slidably housed in the turning cover 84.

The first and second jaws 101, 102 are formed of a rigid body having rigidity equal to or greater than that capable of preventing buckling of the first and second jaws 101, 102 when the first and second jaws 101, 102 are inserted into a living body and caused to make a turning manipulation or an opening and closing manipulation, thereby treating a living tissue. Thus, the first and second jaws 101, 102 are formed of a metal material such as a stainless steel material, for example or a hard resin material such as a reinforced plastic material. Heater patterns (heating members) 124, 125 described later are arranged at the first and second jaws 101, 102, and thus, these patterns each have heat resistance.

As shown in FIG. 3, the first jaw 101 includes a first jaw main body 110 and a soft receptacle member 111. The receptacle member 111 receives a heat transmission member 114 described later, of the second jaw 102. The receptacle member 111 receives the heat transmission member 114, and thus, is formed of a resin material such as a rubber member having heat resistance.

On the other hand, the second jaw 102 includes a second jaw main body 113, the heat transmission member 114, and a heat insulating member 115. The heat transmission member 114 is provided at a position opposite to the first jaw 101. It is preferable that the heat transmission member 114 be made of a metal material such as molybdenum or copper, for example. The heat insulating member 115 is provided between the second jaw main body 113 and the heat transmission member 114. It is preferable that the heat insulating member 115 be made of PTFE as a resin material having heat resistance. The heat transmission member 114 is fixed to the heat insulating member 115 by means of fixing pins 114a, 114b, for example. The heat insulating member 115 is fixed to the second jaw main body 113 by means of engagement or adhesion, for example.

First and second electrodes 121, 122 are arranged at the heat transmission member 114 of the second jaw 102. The first lead wire 13 is connected to the first electrode 121 by means of soldering, for example. A heater pattern 124 is connected to this first electrode 121. The second lead wire 14 is connected to the second electrode 122 by means of soldering. A heater pattern 125 is connected to this second electrode 122.

Thus, after a power supply device has been connected to a connector 80 shown in FIG. 5, when current is supplied to the electrodes 121, 122 through the first and second lead wires 13, 14 by means of the connector 80, the heater patterns 124, 125 generate heat. The heat is transferred from the heater patterns 124, 125 to the heat transmission member 114. At this time, the heat is efficiently transferred to the heat transmission member 114 by means of the heat insulating member 115 of the second jaw 102. Therefore, a coagulation procedure or an incision procedure can be performed to a living tissue by means of the first and second jaws 101, 102.

A function of the thus formed surgical instrument 1 will be described here.

A surgeon inserts a predetermined finger of his/her right hand, for example, into handle rings 71a, 72a of first and second handles 71, 72. In this manner, the surgeon grips an opening-and-closing manipulating section 4b.

In an initial state, as shown in FIG. 1, a pair of jaws 101, 102 of the surgery section 3 of the surgical instrument 1 are closed and is set in a state in which the axial direction of the surgery section 3 is oriented in the same direction as the axial direction (X-axis direction) of the insertion section 2.

In this state, the opening-and-closing manipulating section 4b is manipulated. As shown in FIGS. 5 and 11, with a handle pivot pin 73 being a fulcrum, the second handle 72 is opened to be turned with respect to the first handle 71. At this time, the ball section 79a is forwardly moved by means of the rotation drive pipe receptacle section 79 of the second handle 72, and the rotation drive pipe 38 is forwardly moved. When the rotation drive pipe 38 is moved forwardly, the first drive member 10 moves forwardly, following the forward movement of the rotation drive pipe 38. Then, as shown in FIGS. 3 and 9, a first coupling pin 88 at the distal end of the first drive member 10 moves forwardly. Thus, as shown in FIGS. 3, 4, 9, and 12, the first link arm 89 moves forwardly. As shown in FIGS. 3 and 9, when the first link arm 89 moves forwardly, the second link arm 91 abutted against two abutment faces 84a, 84b on the inner periphery face of the turning cover 84 moves forwardly in the inside of the turning cover 84.

As shown in FIGS. 4, 6, 10, and 12, a second opening-and-closing pin 107 is supported in an immobile state by means of the turning cover 84 so that the movement in the axial direction of the turning cover 84 of the first jaw 101 is restricted. Thus, as shown in FIGS. 3 and 9, when the second link arm 91 moves forwardly in the inside of the turning cover 84, the first jaw 101 is turned by the second opening-and-closing pin 107. Then, with the first opening and closing pin 105 being a pivot axis, the second jaw 102 turns with respect to the first jaw 101. Therefore, the first and second jaws 101, 102 turn with respect to each other, and the first and second jaws 101, 102 open with respect to each other. Thus, the first and second jaws 101, 102 turn in a direction deviating from the axial direction of the insertion section 2.

As shown in FIGS. 3 and 9, the second drive member 11 is immobile independent of manipulations of the first and second handles 71, 72. Thus, a force for turning the turning cover 84 is not transferred from the distal end of the second drive member 11 to the turning cover 84.

The surgical instrument 1 is turned so that the second handle 72 is spaced from the handle 71 around a handle pivot pin 73 from the initial state shown in FIG. 1. Then, as shown in FIG. 13, the first and second jaws 101, 102 of the surgery section 3 open substantially symmetrically with respect to each other. When a turning quantity (opening and closing quantity) of the second handle is maintained with respect to the first handle 71 in a predetermined range, the first and second jaws 101, 102 open by a quantity corresponding to the turning quantity.

As shown in FIG. 5, a state (initial state) in which the second handle 72 is closed with respect to the first handle 71 is established. The turning manipulating section 4a is operated from this state. As shown in FIGS. 5 and 14, the first rotary knob 31 is gripped, and the first rotary knob 31 is rotated along the axis of the insertion section 2, for example, in the right direction. The rotation of the first rotary knob 31 is converted to a forward motion of the turning base 40 by means of the first and second screw sections 45, 46 (refer to FIG. 14). That is, a state in which the first and second screw sections 45, 46 are spirally fitted acts as motion direction converting means for converting a rotational motion into a forward and backward motion.

When the turning base 40 advances, the drive member fixing pin 41 and the proximal end of the second drive member 11 are coupled with each other so that the second drive member 11 advances to the sheath 20, following the advancement of the turning base 40. At this time, the distal end of the second drive member 11 is coupled with the proximal end of the turning cover 84 by means of the second turning pin 86.

As shown in FIGS. 3, 4, 15, and 16, the first drive member 10 is immobile because the first and second handles 71, 72 are not opened or closed. Thus, a force is applied in a direction in which the turning cover 84 advances by advancement of the second drive member 11.

As shown in FIGS. 3 and 15, the turning cover 84 is pivoted by means of the first turning pins 83a, 83b, whereby forward movement relevant to the insertion section distal end cover 82 is restricted. Thus, while the first turning pins 83a, 83b at the proximal end of the turning cover 84 are defined as pivot axes, turning is established with the inside of the ZX plane being upwardly oriented (refer to FIGS. 6 and 17). At this time, a turning angle is specified by a length in the axial direction of the insertion section 2 of the guide groove 34a. In the present embodiment, a turning angle (turning quantity) of the surgery section 3 is set in the range of 0 degree to 60 degrees. This maximum turning angle can be changed as required.

Therefore, in the surgical instrument 1, when the first rotary knob 31 is rotated around the axis of the insertion section 2 from the initial state shown in FIG. 1, the turning base 40 advances as shown in FIG. 14 and the turning cover 84 turns as shown in FIG. 18. Thus, the entire surgery section 3 turns in the ZX plane at the distal end of the insertion section 2. When the rotation quantity of the first rotary knob 31 is maintained at a predetermined position, a position at which the surgery section 3 has turned is maintained in response to its rotation quantity.

The opening-and-closing manipulating section 4b is manipulated from the state shown in FIG. 18. As shown in FIGS. 14 and 19, the second handle 72 opens while it is turned with respect to the first handle 71. As shown in FIGS. 7, 15, 16, and 20, the first drive member 10 is advanced, and the second link arm 91 is slid forwardly in inside of the turning cover 84 by means of the first link arm 89. At this time, the second link arm 91 obliquely slides along the turning cover 84 due to the advancement of the first drive member 10 because the turning cover 84 is in a turned state. Then, in a state in which the turning cover 84 has been turned with respect to the insertion section 2, the first and second jaws 101, 102 open with respect to each other.

As shown in FIGS. 7, 15, 16, and 20, the second drive member 11 does not depend on turning of the second handle 72, and is immobile because the first rotary knob 31 is immobile. The second drive member 11 maintains a state in which the turning cover 84 has turned with respect to the insertion section 2 (refer to FIGS. 8 and 17).

Therefore, in the surgical instrument 1, when the second handle 72 is turned so as to open with respect to the first handle 71 around the handle pivot pin 73 from the state shown in FIG. 18, the first and second jaws 101, 102 which are distal end tools of the surgery section 3 relatively open as shown in FIG. 21.

When the second handle 72 of the opening-and-closing manipulating section 4b described above is closed after being turned so as to be proximal to the first handle 71, counteraction against the above-described action is caused, and the first and second jaws 101, 102 are closed. When the first rotary knob 31 described above is rotated in the left direction with respect to the axial direction of the insertion section 2, counteraction against the above-described action is caused, and the second drive member 11 is attracted to the manipulating section 4. The surgery section 3 turned with respect to the insertion section 2 returns to a position (refer to FIG. 1) extending straightway to the insertion section 2.

The second rotary knob 32 provided backwardly of the first rotary knob 31 is gripped, and this second rotary knob 32 is rotated around the axis of the insertion section 2, for example, in the right direction. Then, this rotation of the second rotary knob 32 is transferred from the second rotary knob 32 to the second base 35 coupled by the fixing pin 53. This rotation is further transferred from the second base 35 to the first base 34, and then, to the first and second sheaths 21, 22. Then, the second drive member 11 rotates around the axis of the insertion section 2.

On the other hand, when the second base 35 rotates, the rotation drive pipe 38 rotates around the axis of the insertion section 2 from the drive pipe cover member 54 by the flat section 55. Thus, the first drive member 10 engaged with the rotation drive pipe 38 by means of an engagement portion rotates around the axis of the insertion section 2.

When the second rotary knob 32 is rotated around the axis of the insertion section 2, the first and second sheaths 21, 22 and the first and second drive members 10, 11 rotate around the axis of the insertion section 2. Thus, the first and second jaws 101, 102 of the surgery section 3 rotate around the axis of the inserts section 2, following the above rotation.

In this manner, proper surgery is made to a living tissue by combining a turning manipulation of the turning manipulating section 4a and an opening and closing manipulation of the opening-and-closing manipulating section 4b with each other. The illustrated turning angle or opening angle of the surgery section 3 can be stopped in a proper range without being limited to these angles.

The surgical instrument 1 is used when a living tissue is subjected to a coagulation procedure or an incision procedure or when a blood vessel adhering to the living tissue is released from the tissue.

A description will be given with respect to an operation of gripping a living tissue between the first and second jaws 101 and 102, and then, subjecting the gripped living tissue to a coagulation procedure or a incision procedure. Specifically, a surgery of pinching a blood vessel or griping a living tissue itself is carried out. First, the connector 80 at the proximal end of the lead wire 13, 14 is connected to a power supply device.

Next, for example, with an abdominal cavity being inflated by introducing gas, a sheath tube or the like is inserted into a living tissue through the distal end of the surgery section 3. The manipulating section 4 is manipulated, and is approached to a position of a target living tissue by combining turning and rotation of the surgery section 3 described above. By combining the above-described turning and rotation, the manipulating section can be easily approached to a desired position.

At this time, the O-rings 43a, 43b between the first base 34 and the turning base 40 prevent gas from leaking from the second lumen 24. An air tight cap 77 prevents gas from leaking from the first lumen 23.

This surgery is made by closing the first and second jaws 101, 102, after they have been temporarily opened in a state in which the surgery section 3 of the surgical instrument 1 has been turned at an arbitrary angle with respect to the insertion section 2. Specifically, the second handle 72 is turned so as to deviate from the first handle 71, and the first and second jaws 101, 102 are opened with respect to each other. The manipulating section 4 is moved so that the desired positioned living tissue is disposed between the fist and second jaws 101 and 102, the surgery section 3 is turned or rotated in the desired direction, and further, the surgical instrument 1 itself is moved. The second handle 72 is turned so as to be proximal to the first handle 71, and the first and second jaws 101, 102 are closed with respect to each other. At this time, when the turning position of the first turning knob 31 is maintained, the second drive member 11 is immobile with respect to the insertion section 2. Thus, only the first drive member 10 is attracted to the proximal end of the insertion section 2.

A distal end of the first drive member 10 is coupled with a proximal end of the first link arm 89, and a distal end of the first link arm 89 is coupled with a proximal end of the second link arm 91, by means of a member having rigidity. Thus, a force applied to the first drive member 10 is reliably and easily transferred to the second link arm 91 by means of the first link arm 89. Therefore, the living tissue is gripped while the first and second jaws 101, 102 are closed with respect to each other.

The first drive member 10, the first link arm 89, and the second link arm 91 are formed of a rigid body such as a stainless steel material. Thus, even in a state in which the surgery section 3 has turned with respect to the insertion section 2, a force of relatively closing the first and second jaws 101, 102 can be reliably transferred while the turned state is maintained. In this manner, the living tissue or blood vessel can be easily gripped in a state in which the surgery section 3 has turned with respect to the insertion section 2.

In this state, a current is supplied from the power supply device to the connector 80, and power is conducted via the lead wires 13, 14. A current is supplied to the electrodes 121, 122 by means of the lead wires 13, 14. When a current is supplied to the electrodes 121, 122, the heater patterns 124, 125 generate heat. The heat generated by the heater patterns 124, 125 is transferred to the heat transmission member 114, and the gripped living tissue or blood vessel is subjected to a coagulation procedure or an incision procedure.

At this time, the heat insulating member 115 is arranged between the heat transmission member 114 and the second jaw main body 113 so that the heat generated by the heater patterns 124, 125 is prevented from being transferred to the second jaw main body 113. Thus, the heat generated by the heater patterns 124, 125 is efficiently supplied to the heat transmission member 114. The second jaw main body 113 prevents the living tissue from being subjected to the coagulation procedure or incision procedure.

Now, a description will be given with an operation of making a surgery for releasing a blood vessel adhering to a living tissue from the tissue.

This surgery is made by releasing the first and second jaws 101, 102 from their closed state (refer to FIG. 21) in a state in which the surgery section 3 of the surgical instrument 1 has been turned at an arbitrary angle with respect to the insertion section 2, for example, (refer to FIG. 18). As shown in FIGS. 14 and 19, the second handle 72 is turned so as to deviate from the first handle 71, and then, the first and second jaws 101, 102 are opened with respect to each other. The second link arm 91 moves along the axial direction of the turning cover 84. Thus, even in a state in which the surgery section 3 has turned with respect to the insertion section 2, a release force can be reliably transferred to the first and second jaws 101, 102 while the turned state is maintained. By doing thus, the blood vessel adhering to a living tissue can be easily released.

When a push button 64 of the manipulating section 4 is pushed downwardly along the Z-axis direction shown in FIG. 5, for example, a second engagement member 62 is moved downwardly along the Z-axis direction by means of an engagement pin 63. In this manner, the first and second engagement members 60 and 62 are disengaged from each other. At this time, the second rotary knob 32 is moved in a direction of the distal end of the insertion section 2 with respect to the third base 36. By doing thus, the turning manipulating section 4a and the opening-and-closing manipulating section 4b can be separated from each other. Thus, the surgical instrument 1 can be easily washed completely.

As has been described above, the surgical instrument 1 according to the present embodiment is featured as follows.

The first and second drive members 10, 11, the first and second link arms 89, 91, the distal end cover 82, the turning cover 84, and the sheath 20 or the like are formed of a member having rigidity. The second lumen 24 is formed as a region of size which is substantially equal to thickness of the second drive member 11 whose transverse section is formed in a rectangular shape. Thus, even a member such as the second drive member 11 which is comparatively thin and extends in a longitudinal direction of the insertion section 2 is prevented from buckling, and an axial force transferred to the second drive member 11 can be reliably transferred to the turning cover 84.

In this manner, when a proper surgery is performed to a living tissue, no matter what turning posture the surgery section 3 is set with respect to the insertion section 2, a desired turning posture can be maintained. No matter what open or closed state the first and second jaws 101, 102 which are distal end tools of the surgery section 2 take, a desired turning posture can be maintained. Therefore, by opening and closing the distal end tool, the distal end tool can be turned with respect to the insertion section 2 in a state in which a living tissue has been gripped.

When the second rotary knob 32 is manipulated to rotate the surgery section 3, the distal end tool can be rotated in a desired direction up to a desired position. The opening and closing direction of the distal end tool can be properly adjusted to be a desired direction by a surgeon. In this manner, in a state in which the distal end tool is opened and closed, and then, a living tissue is gripped by the distal end tool, the distal end tool can be rotated or turned in a desired direction.

Even if an external force for turning the surgery section 3 is directly acted on the surgery section 3, the section itself does not extend since the second drive member 11 has rigidity, and there occurs no buckling since the section is accommodated in the second lumen 24 having rigidity. Thus, the turning posture of the surgery section 3 can be prevented from being changed unintentionally.

In a state in which a living tissue in a desired position is approached by combination of turn and rotation of the surgery section 3, and the living tissue is gripped by the distal end tool, current is supplied from the power supply device to the electrodes 121, 122 via the lead wires 13, 14. Then, the heater patterns 124, 125 generate heat, and this heat is transferred to the living tissue via the heat transmission member 114. Thus, a desired site of the living tissue can be approached easily, and a variety of surgery such as a coagulation procedure or an incision procedure of the living tissue can be made with respect to the approached position. At this time, the heat transmission member 114 is covered with the heat insulating member 115 so that the heat generated by the heater patterns 124, 125 can be prevented from being transferred to the second jaw main body 113.

As described above, no matter what opened and closed state the distal end tool of the surgery section 3 takes, a desired turning posture can be maintained. Thus, the blood vessel adhering to the living tissue, for example, can be released by using the outer periphery face of the first and second jaws 101, 102.

Therefore, the turning manipulating section 4a of the manipulating section 4 is thus manipulated, whereby a desired site of the living tissue can be easily approached by combining rotation and turning of the surgery section 3. The opening-and-closing manipulating section 4b of the manipulating section 4 is manipulated, and the living tissue is gripped by means of the distal end tool, whereby the gripped living tissue can be subjected to a surgery such as a coagulation procedure or an incision procedure. The opening-and-closing manipulating section 4b is manipulated, whereby the adhering living tissue can be released from each other by means of the distal end tool.

That is, according to the present embodiment, no matter what turning posture the surgery section 3 takes with respect to the inserts section 2, the distal end tool can be maintained at an arbitrary opening and closing angle while it has a sufficient opening and closing force. Power is supplied to the heater patterns 124, 125 provided at the distal end tool so that the living tissue can be subjected to a surgery such as a coagulation procedure or an incision procedure. No matter what turning posture the surgery section 3 takes with respect to the insertion section 2, the distal end tool is opened in a state in which the turned state of the surgery section 3 is maintained, and the adhering living tissue can be released. In this way, when a proper surgery is performed to the living tissue, the surgery section 3 or the insertion section 2 is prevented from buckling or the surgery section 3 is prevented from turning with respect to the insertion section 2 while it cannot bear an external force applied by the living tissue.

The first and second drive members 10, 11 of the insertion section 2 have rigidity, respectively. Thus, the turning manipulating section 4a and the opening-and-closing manipulating section 4b of the manipulating section 4 are manipulated quickly by one hand, whereby a motive force can be efficiently transferred. Thus, the surgery section 3 can be turned with respect to the insertion section 2 quickly or rotated around the axis of the insertion section 2, or can make an opening and closing operation of the first and second jaws 101, 102 which are distal end tools of the surgery section 3.

Irregularities are provided on grip faces 101a, 102a of a pair of jaws 101, 102, respectively. Thus, a living tissue which is a grip target or a suture needle and a suture thread or the like can be reliably gripped.

The surgery section 3 can be rotated together with the insertion section 2 with respect to the opening-and-closing manipulating section 4b of the manipulating section 4. Thus, the orientations of the first and second jaws 101, 102 can be changed. In this manner, a living tissue whose position is hardly approached can be easily treated.

The distal end tool is not limited to the first and second jaws 101, 102 described previously. For example, it is preferable that the tool be properly formed in a variety of shapes such as release forceps or grip forceps.

The present embodiment has described that abutment faces 84a, 84b are provided on an inner periphery face of the turning cover 84, and the second link arm 91 is retractable in parallel to the turning cover 84. Without being limited thereto, a pair of guide grooves, for example, are provided on the inner periphery face of the turning cover 84, a pair of guide pins are provided at the second link arm 91, and these guide pins are disposed in the guide groves, whereby the second link arm 91 may be provided in parallel to the turning cover 84.

The present embodiment has described that the second drive member 11 advances when the first rotary knob 31 is turned in the right direction with respect to the axial direction of the insertion section 2. Without being limited thereto, the second drive member 11 may be configured to advance when the first rotary knob 31 is turned in the left direction with respect to the axial direction of the insertion section 2. In order for the second drive member 11 to move in an forward direction, a direction in which the first rotary knob 31 is rotated can be changed depending on how the first and second screw sections 45, 46 are provided, as required.

Therefore, with the surgical instrument 1 according to the present embodiment, the surgery section 3 has rigidity capable of maintaining a turned state, and further, the distal end tool can be opened and closed while it has a sufficient opening and closing force no matter what posture the surgery section 3 takes in the range of 0 degree to 60 degrees with respect to the insertion section 2, thus making it possible to improve operability and practicability while in actual use. While the surgery section 3 is in a turned state with respect to the insertion section 2, it is possible to subject a tissue to a coagulation procedure or an incision procedure. Thus, every site can be easily approached, and the approached living tissue can be treated.

Up to now, one embodiment has been specifically described with reference to the accompanying drawings. The present invention is not limited to the above-described embodiment and includes all embodiments made without deviating from the scope of the invention as defined by the claims.

### Industrial Applicability

According to the present invention, there can be provided a surgical instrument capable of easily approaching a desired site of a living tissue and capable of subjecting the living tissue to a coagulation procedure or an incision procedure.

## Claims

1. A surgical instrument (1) comprising:
a first sheath (21) having a distal end portion, a proximal end portion and a central axis extended from the distal end portion to the proximal end portion in a longitudinal direction;
a pair of grip sections (101, 102) which are provided at the distal end portion of the first sheath and which are configured to relatively open and close; and
a turning section (84) which is connected between the first sheath and the grip sections, which is configured to turn an orientation of the grip sections in a direction deviating from the central axis of the first sheath;
an opening-and-closing manipulating section (4b) which is provided at the proximal end portion of the first section and which is configured to open and close the grip sections;
a turning manipulating section (4a) which is configured to turn the turning section;
a heating member (124, 125) which is arranged in at least one of the grip sections and which is configured to generate heat by power supply,
a second sheath (22) inserted into the first sheath;
an opening-and-closing drive shaft (10) which is inserted into the second sheath, which is connected to the opening-and-closing manipulation section (4b) and which is configured to open and close the grip sections together with operation of the opening-and-closing manipulation section; and
a turning drive shaft (11) which is provided between the first sheath and the second sheath parallel with the longitudinal direction, which is connected to the turning manipulating section (4a) and which is configured to turn the turning section with respect to the longitudinal direction of the first sheath together with operation of the turning manipulation section.

2. The surgical instrument (1) according to claim 1, **characterized by** further comprising:
a turning drive mechanism (11, 34, 40, 41, 86) including the turning drive shaft (11), the turning drive mechanism being provided between the turning section (84) and the turning manipulating section (4a) and configured to turn the turning section with respect to the longitudinal direction of the first sheath together with operation of the turning manipulating section; and
an opening-and-closing drive mechanism (10, 38, 79, 89, 91, 105, 107) including the opening-and-closing drive shaft (10), the opening-and-closing drive mechanism being provided between the grip sections (101, 102) and the opening-and-closing manipulating section (4b) and configured to open and close the grip sections together with operation of the opening-and-closing manipulating section.

3. The surgical instrument (1) according to claim 2, **characterized in that**
the opening-and-closing drive shaft (10) includes a first drive member (10) having a distal end portion and a proximal end portion, the first drive member being arranged in the first sheath (21) so as to be movable along the longitudinal direction of the first sheath,
the opening-and-closing drive mechanism (10, 38, 79, 89, 91, 105, 107) comprises:
an opening and closing operation force transfer mechanism (38, 79) which is provided between the proximal end portion of the first drive member and the opening-and-closing manipulating section (4b) and which is configured to convert a manipulating force of the opening-and-closing manipulating section into a movement force of the first drive member; and
an opening and closing mechanism (89, 91, 105, 107) which is provided between the distal end portion of the first drive member and the grip section (101, 102) and which is configured to open and close the grip sections supported by the turning section (84) by movement of the first drive member.

4. The surgical instrument (1) according to claim 3, **characterized in that** the first drive member (10) is formed of a rigid member.

5. The surgical instrument (1) according to claim 3 or 4, **characterized in that** the first drive member (10) comprises:
an inner hole (10a) formed along the central axis of the first sheath (21); and
a transmission member (13, 14) which is inserted through the inner hole and which is configured to supply electric energy to the heating member (124, 125).

6. The surgical instrument (1) according to claim 2, **characterized in that** the turning drive mechanism (11, 34, 40, 41, 86) comprises:
a second drive member (11) having a distal end portion and a proximal end portion, the second drive member arranged in the first sheath (21) so as to be movable along the longitudinal direction of the first sheath;
a turning operation force transfer mechanism (34, 40, 41) which is provided between the proximal end portion of the second drive member and the turning manipulating section (4a) and which is configured to convert a manipulating force of the turning manipulating section into a movement force of the second drive member; and
a turning mechanism (86) which is provided between the distal end portion of the second drive member and the turning section (84) and which is configured to move the turning section in a direction deviating from the central axis of the first sheath by movement of the second drive member.

7. The surgical instrument (1) according to claim 6, **characterized in that** the second drive member (11) is formed of a rigid member.

8. The surgical instrument (1) according to claim 1, **characterized in that**
the first sheath (21) is formed in a cylindrical shape and whose section perpendicular to the central axis is hollow.
the second sheath (22) is formed in a cylindrical shape and whose section perpendicular to the central axis has a flat shape bulkhead (22a),
the first sheath includes a first region (23) which is an internal space of the second sheath and a second region (24) which is a space between the first sheath and the second sheath,
at least part of the opening-and-closing drive mechanism (10, 38, 79, 89, 91, 105, 107) is housed in the first region (23), and
at least part of the turning drive mechanism (11, 34, 40, 41, 86) is housed in the second region (24).

9. The surgical instrument (1) according to claim 8, **characterized in that**
the bulkhead (22a) includes a first flat section which is in parallel to the longitudinal direction of the first sheath (21),
the second drive member (11) includes a second flat section which is in parallel to the first flat section,
the first flat section and the second flat section are brought into contact with each other so as to enable the second drive member (11) to slide in the longitudinal direction of the first sheath (21), and a position opposite to the second flat section of the second drive member is supported in the second region.

10. The surgical instrument (1) according to claim 2, **characterized in that** the turning manipulating section (4a) comprises:
a turning manipulating knob (31) which is provided at the proximal end portion of the first sheath (21) so as to enable rotating operation and which is configured to turn the turning section (84) at the distal end portion of the first sheath (21) with respect to the longitudinal direction by the turning drive mechanism (11, 34, 40, 41, 86); and
a rotating manipulating knob (32) which is provided at the proximal end portion of the first sheath (21) to enable rotating operation and which is configured to rotate the first sheath, the turning section, the turning drive mechanism, and the opening-and-closing drive mechanism (10, 38, 79, 89, 91, 105, 107) around the central axis of the first sheath, and
the opening-and-closing manipulating section (4b) includes an opening and closing manipulating handle (71, 72) manipulated so as to open and close the grip sections (101, 102).

11. The surgical instrument (1) according to claim 8, **characterized in that**
the opening-and-closing drive shaft (10) includes a first rigid drive bar (10) which extends in the first region (23),
the first drive bar is formed in a cylindrical shape and includes a third region (10a) which houses a transfer member (13, 14) which is configured to transfer electric energy to the heating member (124, 125) on the central axis of the first sheath (21), and
the turning drive shaft (11) includes a second rigid drive bar (11) which extends in the second region (24).

12. The surgical instrument (1) according to claim 8, **characterized in that** an effective traverse sectional area of the second region (24) is configured to be smaller than an effective traverse sectional area of the first region (23).

## Patentansprüche

1. Chirurgisches Instrument (1), das umfasst:
einen ersten Mantel (21) mit einem distalen Endabschnitt, einem proximalen Endabschnitt und einer Mittelachse, die sich vom distalen Endabschnitt zum proximalen Endabschnitt in Längsrichtung erstreckt;
ein Paar von Greifabschnitten (101, 102), die am distalen Endabschnitt des ersten Mantels bereitgestellt sind und die so konfiguriert sind, dass sie sich relativ öffnen und schließen; und
einen Drehabschnitt (84), der zwischen dem ersten Mantel und den Greifabschnitten verbunden ist, der so konfiguriert ist, dass er eine Ausrichtung der Greifabschnitte in eine Richtung dreht, die von der Mittelachse des ersten Mantels abweicht;
einen Öffnen-und-Schließen-Manipulationsabschnitt (4b), der am proximalen Endabschnitt des ersten Abschnitts bereitgestellt ist und der so konfiguriert ist, dass er die Greifabschnitte öffnet und schließt;
einen Drehmanipulationsabschnitt (4a), der so konfiguriert ist, dass er den Drehabschnitt dreht;
ein Heizelement (124, 125), das in zumindest einem der Greifabschnitte angeordnet ist und das so konfiguriert ist, dass es Wärme durch eine Energieversorgung erzeugt,
einen zweiten Mantel (22), der in den ersten Mantel eingesetzt ist;
eine Öffnen-und-Schließen-Antriebswelle (10), die in den zweiten Mantel eingesetzt ist, die mit dem Öffnen-und-Schließen-Manipulationsabschnitt (4b) verbunden ist und die so konfiguriert ist, dass sie die Greifabschnitte gemeinsam mit dem Betrieb des Öffnen-und-Schließen-Manipulationsabschnitts öffnet und schließt; und
eine Drehantriebswelle (11), die zwischen dem ersten Mantel und dem zweiten Mantel parallel zur Längsrichtung bereitgestellt ist, die mit dem Drehmanipulationsabschnitt (4a) verbunden ist und die so konfiguriert ist, dass sie den Drehabschnitt in Bezug auf die Längsrichtung des ersten Mantels gemeinsam mit dem Betrieb des Drehmanipulationsabschnitts dreht.

2. Chirurgisches Instrument (1) nach Anspruch 1, **dadurch gekennzeichnet, dass** es ferner umfasst:
einen Drehantriebsmechanismus (11, 34, 40, 41, 86), der die Drehantriebswelle (11) umfasst, wobei der Drehantriebsmechanismus zwischen dem Drehabschnitt (84) und dem Drehmanipulationsabschnitt (4a) bereitgestellt und so konfiguriert ist, dass sie den Drehabschnitt in Bezug auf die Längsrichtung des ersten Mantels gemeinsam mit dem Betrieb des Drehmanipulationsabschnitts dreht; und
einen Öffnen-und-Schließen-Antriebsmechanismus (10, 38, 79, 89, 91, 105, 107), der die Öffnen-und-Schließen-Antriebswelle (10) umfasst, wobei der Öffnen- und-Schließen-Antriebsmechanismus zwischen den Greifabschnitten (101, 102) und dem Öffnen-und-Schließen-Manipulationsabschnitt (4b) bereitgestellt und so konfiguriert ist, dass er die Greifabschnitte gemeinsam mit dem Betrieb des Öffnen- und-Schließen-Manipulationsabschnitt öffnet und schließt.

3. Chirurgisches Instrument (1) nach Anspruch 2, **dadurch gekennzeichnet, dass**:
die Öffnen-und-Schließen-Antriebswelle (10) ein erstes Antriebselement (10) mit einem distalen Endabschnitt und einem proximalen Endabschnitt umfasst, wobei das erste Antriebselement im ersten Mantel (21) angeordnet ist, um entlang der Längsrichtung des ersten Mantels beweglich zu sein,
der Öffnen-und-Schließen-Antriebsmechanismus (10, 38, 79, 89, 91, 105, 107) umfasst:
einen Öffnen-und-Schließen-Betrieb-Kraftübertragungsmechanismus (38, 79), der zwischen dem proximalen Endabschnitt des ersten Antriebselements und dem Öffnen-und-Schließen-Manipulationsabschnitt (4b) bereitgestellt ist und der so konfiguriert ist, dass er eine Manipulationskraft des Öffnen-und-Schließen-Manipulationsabschnitts in eine Bewegungskraft des ersten Antriebselements umwandelt; und
einen Öffnen-und-Schließen-Mechanismus (89, 91, 105, 107), der zwischen dem distalen Endabschnitt des ersten Antriebselements und dem Greifabschnitt (101, 102) bereitgestellt ist und der so konfiguriert ist, dass er die Greifabschnitte, die vom Drehabschnitt (84) getragen werden, durch Bewegung des ersten Antriebselements öffnet und schließt.

4. Chirurgisches Element (1) nach Anspruch 3, **dadurch gekennzeichnet, dass** das erste Antriebselement (10) durch ein steifes Element gebildet ist.

5. Chirurgisches Element (1) nach Anspruch 3 oder 4, **dadurch gekennzeichnet, dass** das erste Antriebselement (10) umfasst:
ein Innenloch (10a), das entlang der Mittelachse des ersten Mantels (21) gebildet ist; und
ein Übertragungselement (13, 14), das durch das innere Loch eingesetzt ist und das so konfiguriert ist, dass es das Heizelement (124, 125) mit elektrischer Energie versorgt.

6. Chirurgisches Element (1) nach Anspruch 2, **dadurch gekennzeichnet, dass** der Drehantriebsmechanismus (11, 34, 40, 41, 86) umfasst:
ein zweites Antriebselement (11) mit einem distalen Endabschnitt und einem proximalen Endabschnitt, wobei das zweite Antriebselement im ersten Mantel (21) angeordnet ist, um entlang der Längsrichtung des ersten Mantels beweglich zu sein,
einen Drehbetrieb-Kraftübertragungsmechanismus (34, 40, 41), der zwischen dem proximalen Endabschnitt des zweiten Antriebselements und dem Drehmanipulationsabschnitt (4b) bereitgestellt ist und der so konfiguriert ist, dass er eine Manipulationskraft des Drehmanipulationsabschnitts in eine Bewegungskraft des zweiten Antriebselements umwandelt; und
einen Drehmechanismus (86), der zwischen dem distalen Endabschnitt des zweiten Antriebselements und dem Drehabschnitt (84) bereitgestellt ist und der so konfiguriert ist, dass er den Drehabschnitt in eine Richtung, die von der Mittelachse des ersten Mantels abweicht, durch Bewegung des zweiten Antriebselements bewegt.

7. Chirurgisches Element (1) nach Anspruch 6, **dadurch gekennzeichnet, dass** das zweite Antriebselement (11) durch ein steifes Element gebildet ist.

8. Chirurgisches Element (1) nach Anspruch 1, **dadurch gekennzeichnet, dass**:
der erste Mantel (21) in zylindrischer Form ausgebildet ist und sein Schnitt lotrecht zur Mittelachse hohl ist,
der zweite Mantel (22) in zylindrischer Form ausgebildet ist und sein Schnitt lotrecht zur Mittelachse einen flachen Schott (22a) aufweist,
der erste Mantel einen ersten Bereich (23), der ein Innenraum des zweiten Mantels ist, und einen zweiten Bereich (24), der ein Raum zwischen dem ersten Mantel und dem zweiten Mantel ist, umfasst,
zumindest ein Teil des Öffnen-und-Schließen-Antriebsmechanismus (10, 38, 79, 89, 91, 105, 107) im ersten Bereich (23) aufgenommen ist, und
zumindest ein Teil des Drehantriebsmechanismus (11, 34, 40, 41, 86) im zweiten Bereich (24) aufgenommen ist.

9. Chirurgisches Instrument (1) nach Anspruch 8, **dadurch gekennzeichnet, dass**:
der Schott (22a) einen ersten flachen Abschnitt umfasst, der parallel zur Längsrichtung des ersten Mantels (21) verläuft,
das zweite Antriebselement (11) einen zweiten flachen Abschnitt umfasst, der parallel zum ersten flachen Abschnitt verläuft,
der erste flache Abschnitt und der zweite flache Abschnitt miteinander in Kontakt gebracht werden, um zu ermöglichen, dass das zweite Antriebselement (11) in Längsrichtung des ersten Mantels (21) gleitet und eine Position gegenüber dem zweiten flachen Abschnitt des zweiten Antriebselements im zweiten Bereich getragen wird.

10. Chirurgisches Element (1) nach Anspruch 2, **dadurch gekennzeichnet, dass** der Drehmanipulationsabschnitt (4a) umfasst:
einen Drehmanipulationsknopf (31), der am proximalen Endabschnitt des ersten Mantels (21) bereitgestellt ist, um einen Rotationsbetrieb zu ermöglichen, und der so konfiguriert ist, dass er den Drehabschnitt (84) am distalen Endabschnitt des ersten Mantels (21) in Bezug auf die Längsrichtung durch den Drehantriebsmechanismus (11, 34, 40, 41, 86) dreht; und
einen Rotationsmanipulationsknopf (32), der am proximalen Endabschnitt des ersten Mantels (21) bereitgestellt ist, um einen Rotationsbetrieb zu ermöglichen, und der so konfiguriert ist, dass er den ersten Mantel, den Drehabschnitt, den Drehantriebsmechanismus und den Öffnen-und-Schließen-Antriebsmechanismus (10, 38, 79, 89, 91, 105, 107) um die Mittelachse des ersten Mantels rotiert, und
der Öffnen-und-Schließen-Manipulationsabschnitt (4b) einen Öffnen-und-Schließen-Manipulationsgriff (71, 72) umfasst, der manipuliert wird, um die Greifabschnitte (101, 102) zu öffnen und zu schließen.

11. Chirurgisches Instrument (1) nach Anspruch 8, **dadurch gekennzeichnet, dass**:
die Öffnen-und-Schließen-Antriebswelle (10) eine erste steife Antriebsstange (10) umfasst, die sich in den ersten Bereich (23) erstreckt,
die erste Antriebsstange zylindrisch ausgebildet ist und einen dritten Bereich (10a) umfasst, der ein Übertragungselement (13, 14) aufnimmt, das so konfiguriert ist, dass es elektrische Energie an das Heizelement (124, 125) auf der Mittelachse des ersten Mantels (21) überträgt, und
die Drehantriebswelle (11) eine zweite steife Antriebsstange (11) umfasst, die sich in den zweiten Bereich (24) erstreckt.

12. Chirurgisches Element (1) nach Anspruch 8, **dadurch gekennzeichnet, dass** eine effektive Querschnittsfläche des zweiten Bereichs (24) so konfiguriert ist, dass sie kleiner als eine effektive Querschnittsfläche des ersten Bereichs (23) ist.

## Revendications

1. Instrument chirurgical (1) comprenant:
une première gaine (21) ayant une partie d'extrémité distale, une partie d'extrémité proximale et un axe central s'étendant de la partie d'extrémité distale à la partie d'extrémité proximale dans une direction longitudinale;
une paire de sections de préhension (101, 102) qui sont prévues à la partie d'extrémité distale de la première gaine et qui sont configurées pour s'ouvrir et se fermer l'une par rapport à l'autre; et
une section de rotation (84) qui est reliée entre la première gaine et les sections de préhension, qui est configurée pour tourner une orientation des sections de préhension dans une direction s'écartant de l'axe central de la première gaine ;
une section de manipulation d'ouverture et de fermeture (4b) qui est prévue à la partie d'extrémité proximale de la première section et qui est configurée pour ouvrir et fermer les sections de préhension;
une section de manipulation de rotation (4a) qui est configurée pour tourner la section de rotation ;
un élément chauffant (124, 125) qui est agencé dans au moins une des sections de préhension et qui est configuré pour générer de la chaleur par alimentation électrique,
une seconde gaine (22) introduite dans la première gaine;
un arbre d'entraînement d'ouverture et de fermeture (10) qui est introduit dans la seconde gaine, qui est relié à la section de manipulation d'ouverture et de fermeture (4b) et qui est configuré pour ouvrir et fermer les sections de préhension conjointement avec le fonctionnement de la section de manipulation d'ouverture et de fermeture ; et
un arbre d'entraînement en rotation (11) qui est prévu entre la première gaine et la seconde gaine parallèlement à la direction longitudinale, qui est relié à la section de manipulation de rotation (4a) et qui est configuré pour tourner la section de rotation par rapport à la direction longitudinale de la première gaine conjointement avec le fonctionnement de la section de manipulation de rotation.

2. Instrument chirurgical (1) selon la revendication 1, **caractérisé par le fait qu'**il comprend en outre:
un mécanisme d'entraînement en rotation (11, 34, 40, 41, 86) comprenant l'arbre d'entraînement en rotation (11), le mécanisme d'entraînement en rotation étant prévu entre la section de rotation (84) et la section de manipulation de rotation (4a) et configuré pour tourner la section de rotation par rapport à la direction longitudinale de la première gaine conjointement avec le fonctionnement de la section de manipulation de rotation; et
un mécanisme d'entraînement d'ouverture et de fermeture (10, 38, 79, 89, 91, 105, 107) comprenant l'arbre d'entraînement d'ouverture et de fermeture (10), le mécanisme d'entraînement d'ouverture et de fermeture étant prévu entre les sections de préhension (101, 102) et la section de manipulation d'ouverture et de fermeture (4b) et configuré pour ouvrir et fermer les sections de préhension conjointement avec le fonctionnement de la section de manipulation d'ouverture et de fermeture.

3. Instrument chirurgical (1) selon la revendication 2, **caractérisé par le fait que**:
l'arbre d'entraînement d'ouverture et de fermeture (10) comprend un premier élément d'entraînement (10) ayant une partie d'extrémité distale et une partie d'extrémité proximale, le premier élément d'entraînement étant agencé dans la première gaine (21) de façon à être mobile le long de la direction longitudinale de la première gaine,
le mécanisme d'entraînement d'ouverture et de fermeture (10, 38, 79, 89, 91, 105, 107) comprend :
un mécanisme de transfert de force d'actionnement d'ouverture et de fermeture (38, 79) qui est prévu entre la partie d'extrémité proximale du premier élément d'entraînement et la section de manipulation d'ouverture et de fermeture (4b) et qui est configuré pour convertir une force de manipulation de la section de manipulation d'ouverture et de fermeture en une force de mouvement du premier élément d'entraînement ; et
un mécanisme d'ouverture et de fermeture (89, 91, 105, 107) qui est prévu entre la partie d'extrémité distale du premier élément d'entraînement et la section de préhension (101, 102) et qui est configuré pour ouvrir et fermer les sections de préhension supportées par la section de rotation (84) par mouvement du premier élément d'entraînement.

4. Instrument chirurgical (1) selon la revendication 3, **caractérisé par le fait que** le premier élément d'entraînement (10) est formé d'un élément rigide.

5. Instrument chirurgical (1) selon la revendication 3 ou 4, **caractérisé par le fait que** le premier élément d'entraînement (10) comprend:
un trou interne (10a) formé le long de l'axe central de la première gaine (21); et
un élément de transmission (13, 14) qui est introduit à travers le trou interne et qui est configuré pour fournir de l'énergie électrique à l'élément chauffant (124, 125).

6. Instrument chirurgical (1) selon la revendication 2, **caractérisé par le fait que** le mécanisme d'entraînement en rotation (11, 34, 40, 41, 86) comprend:
un second élément d'entraînement (11) ayant une partie d'extrémité distale et une partie d'extrémité proximale, le second élément d'entraînement étant agencé dans la première gaine (21) de façon à être mobile le long de la direction longitudinale de la première gaine ;
un mécanisme de transfert de force d'actionnement de rotation (34, 40, 41) qui est prévu entre la partie d'extrémité proximale du second élément d'entraînement et la section de manipulation de rotation (4a) et qui est configuré pour convertir une force de manipulation de la section de manipulation de rotation en une force de mouvement du second élément d'entraînement ; et
un mécanisme de rotation (86) qui est prévu entre la partie d'extrémité distale du second élément d'entraînement et la section de rotation (84) et qui est configuré pour déplacer la section de rotation dans une direction s'écartant de l'axe central de la première gaine par mouvement du second élément d'entraînement.

7. Instrument chirurgical (1) selon la revendication 6, **caractérisé par le fait que** le second élément d'entraînement (11) est formé d'un élément rigide.

8. Instrument chirurgical (1) selon la revendication 1, **caractérisé par le fait que**:
la première gaine (21) est formée dans une forme cylindrique et dont une section perpendiculaire à l'axe centrale est creuse,
la seconde gaine (22) est formée dans une forme cylindrique et dont une section perpendiculaire à l'axe central a une paroi de forme plate (22a),
la première gaine comprend une première région (23) qui est un espace interne de la seconde gaine et une deuxième région (24) qui est un espace entre la première gaine et la seconde gaine,
au moins une partie du mécanisme d'entraînement d'ouverture et de fermeture (10, 38, 79, 89, 91, 105, 107) est reçue dans la première région (23), et
au moins une partie du mécanisme d'entraînement en rotation (11, 34, 40, 41, 86) est reçue dans la deuxième région (24).

9. Instrument chirurgical (1) selon la revendication 8, **caractérisé par le fait que**:
la paroi (22a) comprend une première section plate qui est parallèle à la direction longitudinale de la première gaine (21),
le second élément d'entraînement (11) comprend une seconde section plate qui est parallèle à la première section plate,
la première section plate et la seconde section plate sont amenées en contact l'une avec l'autre de façon à permettre au second élément d'entraînement (11) de coulisser dans la direction longitudinale de la première gaine (21), et une position opposée à la seconde section plate du second élément d'entraînement est supportée dans la deuxième région.

10. Instrument chirurgical (1) selon la revendication 2, **caractérisé par le fait que** la section de manipulation de rotation (4a) comprend:
un bouton de manipulation de rotation (31) qui est prévu à la partie d'extrémité proximale de la première gaine (21) de façon à permettre une opération de révolution et qui est configuré pour tourner la section de rotation (84) à la partie d'extrémité distale de la première gaine (21) par rapport à la direction longitudinale par le mécanisme d'entraînement en rotation (11, 34, 40, 41, 86); et
un bouton de manipulation de révolution (32) qui est prévu à la partie d'extrémité proximale de la première gaine (21) pour permettre une opération de révolution et qui est configuré pour faire tourner la première gaine, la section de rotation, le mécanisme d'entraînement en rotation et le mécanisme d'entraînement d'ouverture et de fermeture (10, 38, 79, 89, 91, 105, 107) autour de l'axe central de la première gaine, et
la section de manipulation d'ouverture et de fermeture (4b) comprend une poignée de manipulation d'ouverture et de fermeture (71, 72) manipulée de façon à ouvrir et fermer les sections de préhension (101, 102).

11. Instrument chirurgical (1) selon la revendication 8, **caractérisé par le fait que**:
l'arbre d'entraînement d'ouverture et de fermeture (10) comprend une première barre d'entraînement rigide (10) qui s'étend dans la première région (23),
la première barre d'entraînement est formée dans une forme cylindrique et comprend une troisième région (10a) qui reçoit un élément de transfert (13, 14) qui est configuré pour transférer de l'énergie électrique à l'élément chauffant (124, 125) sur l'axe central de la première gaine (21), et
l'arbre d'entraînement en rotation (11) comprend une seconde barre d'entraînement rigide (11) qui s'étend dans la deuxième région (24).

12. Instrument chirurgical (1) selon la revendication 8, **caractérisé par le fait qu'**une aire de section transversale effective de la deuxième région (24) est configurée pour être plus petite qu'une aire de section transversale effective de la première région (23).
